(19) Europäisches Patentamt
European Patent Office
Office européen des brevets

(11) Veröffentlichungsnummer: **0 359 078 A1**

# (12) EUROPÄISCHE PATENTANMELDUNG

(21) Anmeldenummer: **89116259.6**

(22) Anmeldetag: **02.09.89**

(51) Int. Cl.5: **C07D 403/12 , A01N 43/48 , A01N 43/56**

(30) Priorität: **15.09.88 DE 3831430**

(43) Veröffentlichungstag der Anmeldung: **21.03.90 Patentblatt 90/12**

(84) Benannte Vertragsstaaten: **BE CH DE FR GB IT LI NL**

(71) Anmelder: **BAYER AG**
**D-5090 Leverkusen 1 Bayerwerk(DE)**

(72) Erfinder: **Findeisen, Kurt, Dr.**
**In der Follmühle 10**
**D-5068 Odenthal 2(DE)**
Erfinder: **Jelich, Klaus, Dr.**
**Pahlkestrasse 5**
**D-5600 Wuppertal 1(DE)**
Erfinder: **Hänssler, Gerd, Dr.**
**Am Arenzberg 58a**
**D-5090 Leverkusen 3(DE)**

## (54) Substituierte 4-Heterocyclyloximino-pyrazolin-5-one, Verfahren zu ihrer Herstellung und ihre Verwendung als Schädlingsbekämpfungsmittel.

(57) Substituierte 4-Heterocyclyloximino-pyrazolin-5-one der allgemeinen Formel (I),

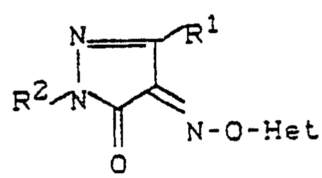

(I)

in welcher
$R^1$, $R^2$ und Het die in der Beschreibung gegebene Bedeutung haben, deren geometrischen Isomeren und Isomerengemische und ihre Verwendung zur Bekämpfung von Schädlingen.

Die neuen Verbindungen sind durch die Formel (I) allgemein definiert, sie können nach Analogieverfahren hergestellt werden, z.B. aus geeigneten 4-Oximino-pyrazolin-5-onen und geeigneten Heterocyclen oder aus geeigneten Alkoximinocarbonsäureestern und geeigneten Hydrazin-Derivaten oder auch aus geeigneten 4-Alkoximino-pyrazolin-5-onen und geeigneten Alkylierungsmitteln.

EP 0 359 078 A1

## Substituierte 4-Heterocyclyloximino-pyrazolin-5-one, Verfahren zu ihrer Herstellung und ihre Verwendung als Schädlingsbekämpfungsmittel

Die Erfindung betrifft neue substituierte 4-Heterocyclyloximino-pyrazolin-5-one, mehrere Verfahren zu ihrer Herstellung und ihre Verwendung als Schädlingsbekämpfungsmittel.

Es ist bekannt, daß bestimmte substituierte Pyrazolinone, wie beispielsweise die Verbindung 4-[(2,6-Dihydroxypyrimidin-4-yl)-methoximino]-1,3-dimethyl-pyrazolin-5-on, fungizide Eigenschaften besitzen (vgl. z.B. EP 212 360).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue substituierte 4-Heterocyclyloximino-pyrazolin-5-one der allgemeinen Formel (I),

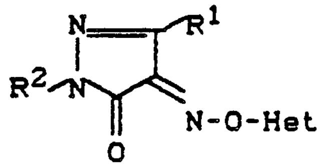

(I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils unsubstituiertes oder jeweils substituiertes Oxiranylalkyl, Aralkyl, Heterocyclyl oder Aryl stehen und

Het für einen unsubstituierten oder substituierten Heterocyclus steht,

gefunden.

Die Verbindungen der Formel (I) können als geometrische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten 4-Heterocyclyloximino-pyrazolin-5-one der allgemeinen Formel (I),

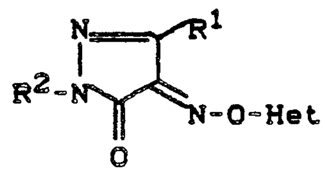

(I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils unsubstituiertes oder jeweils substituiertes Oxiranylalkyl, Aralkyl, Heterocyclyl oder Aryl stehen und

Het für einen unsubstituierten oder substituierten Heterocyclus steht,

erhält, wenn man

(a) 4-Oximino-pyrazolin-5-one der Formel (II),

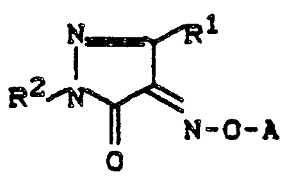

(II)

in welcher

$R^1$ und $R^2$ die oben angegebene Bedeutung haben und

A für Wasserstoff oder ein Alkalimetallkation steht,
mit Heterocyclen der Formel (III),
Het-$E^1$ (III)
in welcher
Het die oben angegebene Bedeutung hat und
$E^1$ für eine elektronenanziehende Abgangsgruppe steht,
gegebenenfalls in Gegenwart eines Verdünnungsmittels, gegebenenfalls in Gegenwart eines Reaktionshilfsmittels und gegebenenfalls in Gegenwart eines Katalysators umsetzt, oder wenn man

(b) Alkoximinocarbonsäureester der Formel (IV),

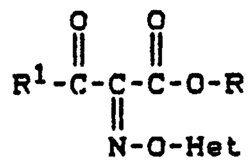

(IV)

in welcher
R für Alkyl steht und
$R^1$ und Het die oben angegebene Bedeutung haben,
mit Hydrazin-Derivaten der Formel (V),
$R^2$-NH-$NH_2$ (V)
in welcher
$R^2$ die oben angegebene Bedeutung hat,
gegebenenfalls in Gegenwart eines Verdünnungsmittels umsetzt,
oder wenn man

(c) die nach Verfahren (a) oder nach Verfahren (b) erhältlichen 4-Alkoximino-pyrazolin-5-one der Formel (Ia),

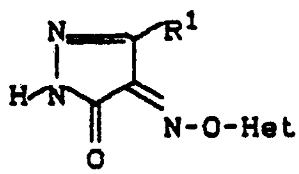

(Ia)

in welcher
$R^1$ und Het die oben angegebene Bedeutung haben,
mit Alkylierungsmitteln der Formel (VI),
$R^2$-$E^2$ (VI)
in welcher
$R^2$ für Alkyl, Alkenyl, Alkinyl, Cyanalkyl, Hydroxyalkyl, Alkoxyalkyl, Alkylthioalkyl, Hydroxycarbonylalkyl, Alkoxycarbonylalkyl oder für jeweils unsubstituiertes oder jeweils substituiertes Aralkyl oder Heterocyclyl steht und
$E^2$ für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen substituierten 4-Heterocyclyloximino-pyrazolin-5-one der Formel (I) eine gute Wirksamkeit gegen Schädlinge besitzen.

Überraschenderweise zeigen die neuen substituierten 4-Heterocyclyloximino-pyrazolin-5-one der Formel (I) u.a. bessere fungizide Eigenschaften als die aus dem Stand der Technik bekannten substituierten Pyrazolinone, wie beispielsweise die Verbindung 4-[(2,6-Dihydroxypyrimidin-4-yl)-methoximino]-1,3-dimethyl-pyrazolin-5-on, welche chemisch und wirkungsmäßig naheliegende Verbindungen sind.

Alkyl in den allgemeinen Formeln bedeutet geradkettiges oder verzweigtes Alkyl, bevorzugt mit 1 bis 8, besonders bevorzugt mit 1 bis 4 und ganz besonders bevorzugt mit 1 bis 3 Kohlenstoffatomen. Beispielhaft seien genannt: Methyl, Ethyl, n- und i-Propyl, n-, s-, i- und t-Butyl, n-Pentyl, i-Pentyl, n-Hexyl, i-Hexyl, n-Heptyl, i-Heptyl, n-Octyl und i-Octyl.

Alkenyl in den allgemeinen Formeln bedeutet geradkettiges oder verzweigtes Alkenyl, bevorzugt mit 2

bis 8 und besonders bevorzugt mit 2 bis 4 Kohlenstoffatomen. Beispielhaft und vorzugsweise seien genannt: Ethenyl, Propenyl-(1), Propenyl-(2), Butenyl-(1), Butenyl-(2) und Butenyl-(3).

Alkinyl in den allgemeinen Formeln bedeutet geradkettiges oder verzweigtes Alkinyl, bevorzugt mit 2 bis 8 und besonders bevorzugt mit 3 oder 4 Kohlenstoffatomen. Beispielhaft seien genannt: Ethinyl, Propinyl-(1), Propinyl-(2) und Butinyl-(3).

Cyanalkyl in den allgemeinen Formeln bedeutet geradkettiges oder verzweigtes Cyanalkyl, bevorzugt mit 1 bis 8 und besonders bevorzugt mit 1 oder 2 Kohlenstoffatomen im Alkylteil. Beispielhaft seien genannt: Cyanmethyl, Cyanethyl, Cyan-n-propyl, Cyan-n-butyl und 2-Cyan-t-butyl.

Hydroxyalkyl in den allgemeinen Formeln bedeutet geradkettiges oder verzweigtes Hydroxyalkyl, bevorzugt mit 1 bis 8, besonders bevorzugt mit 1 oder 2 und ganz besonders bevorzugt mit 2 Kohlenstoffatomen. Beispielhaft seien genannt: Hydroxymethyl, Hydroxyethyl, Hydroxy-n-propyl, Hydroxy-n-butyl und Hydroxy-n-pentyl.

Alkoxyalkyl und Alkylthioalkyl in den allgemeinen Formeln bedeuten in den verschiedenen Alk.-Teilen geradkettiges oder verzweigtes Alkoxyalkyl und Alkylthioalkyl, bevorzugt mit 1 bis 8, besonders bevorzugt mit 1 oder 2 und ganz besonders bevorzugt mit 1 Kohlenstoffatom je Alk.-Teil. Beispielhaft seien genannt: Methoxymethyl, Ethoxymethyl, n-Propoxymethyl, i-Propoxymethyl, n-Butoxymethyl, Methoxyethyl, Ethoxyethyl, n-Propoxyethyl, i-Propoxyethyl, n-Butoxyethyl, Methoxy-n-propyl, Ethoxy-n-propyl, n-Propoxy-n-propyl, i-Propoxy-n-propyl, Methoxy-n-butyl, Methylthiomethyl, Ethylthiomethyl, n-Propylthiomethyl, i-Propylthiomethyl, n-Butylthiomethyl, t-Butylthiomethyl, Methylthioethyl, Ethylthioethyl, n-Propylthioethyl, i-Propylthioethyl, Methylthio-n-propyl, Ethylthio-n-propyl, n-Propylthio-n-propyl, n-Butylthio-n-propyl und t-Butylthio-n-propyl.

Alkoxycarbonyl in den allgemeinen Formeln bedeutet geradkettiges oder verzweigtes Alkoxycarbonyl, bevorzugt mit 1 bis 8 und besonders bevorzugt mit 1 oder 2 Kohlenstoffatomen im Alkoxyteil. Beispielhaft seien genannt: Methoxycarbonyl, Ethoxycarbonyl, n-Propoxycarbonyl, i-Butoxycarbonyl, t-Butoxycarbonyl, i-Butoxycarbonyl, n-Pentoxycarbonyl, i-Pentoxycarbonyl und n-Hexoxycarbonyl.

Hydroxycarbonylalkyl und Alkoxycarbonylalkyl stehen in den allgemeinen Formeln für im Alkyl- und Alkoxyteil geradkettiges oder verzweigtes Hydroxycarbonylalkyl und Alkoxycarbonylalkyl, bevorzugt mit 1 bis 8 und besonders bevorzugt mit 1 oder 2 Kohlenstoffatomen je Alk.-Rest. Beispielhaft seien genannt: Hydroxycarbonylmethyl, Hydroxycarbonylethyl, Hydroxycarbonyl-n-propyl, Hydroxycarbonyl-i-propyl, Hydroxycarbonyl-n-butyl, Methoxycarbonylmethyl, Ethoxycarbonylmethyl, n-Propoxycarbonylmethyl, i-Propoxycarbonylmethyl, n-Butoxycarbonylmethyl, t-Butoxycarbonylmethyl, Ethoxycarbonylethyl, n-Propoxycarbonylethyl, i-Propoxycarbonylethyl, i- Propoxycarbonylethyl, n-Butoxycarbonylethyl, t-Butoxycarbonylethyl, Methoxycarbonyl-n-propyl, Ethoxycarbonyl-n-propyl, n-Propoxycarbonyl-n-propyl und t-Butoxycarbonyl-n-propyl.

Aminocarbonylalkyl, Alkylaminocarbonylalkyl und Dialkylaminocarbonylalkyl stehen in den allgemeinen Formeln für die angegebenen Reste, wobei die Alkylreste geradkettig oder verzweigt sind und bevorzugt jeder Rest 1 bis 8, besonders bevorzugt 1 oder 2 Kohlenstoffatome haben. Beispielhaft seien genannt: Aminocarbonylmethyl, Aminocarbonylethyl, Aminocarbonyl-n-propyl, Aminocarbonyl-i-propyl, Aminocarbonyl-n-butyl, Aminocarbonyl-n-pentyl, Methylaminocarbonylmethyl, Ethylaminocarbonylmethyl, n-Propylaminocarbonylmethyl, i-Propyl- aminocarbonylmethyl, n-Butylaminocarbonylmethyl, Methylaminocarbonylethyl, Ethylaminocarbonylethyl, n-Propylaminocarbonylethyl, i-Propylaminocarbonylethyl, n-Butylaminocarbonylethyl, Methylaminocarbonyl-n-propyl, Ethylaminocarbonyl-n-propyl, n-Propylaminocarbonyl-n-propyl, t-Butylaminocarbonyl-n-propyl, Dimethylaminocarbonylmethyl, Diethylaminocarbonylmethyl, Di-n-propylaminocarbonylmethyl, Dimethylaminocarbonylethyl, Diethylaminocarbonylethyl, Di-n-propylaminocarbonylethyl, Dimethylaminocarbonyl-n-propyl, Diethylaminocarbonyl-n-propyl und Diethylaminocarbonyl-n-butyl.

Oxiranylalkyl in den allgemeinen Formeln bedeutet einen Oxiranylring über einen geradkettigen oder verzweigten Alkylrest, bevorzugt mit 1 bis 4, besonders bevorzugt mit 1 oder 2 Kohlenstoffatomen gebundenen Rest. Beispielhaft seien genannt: Oxiranylmethyl, Oxiranylethyl, Oxiranyl-n-propyl und Oxiranyl-n-butyl.

Aralkyl in den allgemeinen Formeln bedeutet einen im Alkylteil geradkettigen oder verzweigten Aralkylrest bevorzugt mit 6 bis 10 und besonders bevorzugt mit 6 Kohlenstoffatomen im Arylrest und 1 bis 4, besonders bevorzugt mit 1 oder 2 Kohlenstoffatomen im Alkylrest. Der Arylrest bedeutet Naphthyl oder Phenyl, bevorzugt Phenyl.

Aryl hat die oben angegebene Bedeutung in den allgemeinen Formeln.

Heterocyclyl in den allgemeinen Formeln in den Definitionen von $R^1$ und $R^2$ bedeutet einen gesättigten oder ungesättigten Rest mit 5 bis 7 Ringgliedern, bevorzugt mit 5 Ringgliedern und 1 bis 3, bevorzugt 1 Heteroatom, wie Stickstoff, Sauerstoff oder Schwefel oder die Sulfonyl-Gruppierung, bevorzugt Schwefel oder Sulfonyl. Beispielhaft seien genannt: Thienyl, Tetrahydrothienyl und 1,1-Dioxotetrahydrothienyl.

Het in der allgemeinen Formel steht für einen gesättigten oder ungesättigten Ring mit 5 bis 7, bevorzugt 5 oder 6 Ringgliedern, wovon 1 bis 4, bevorzugt 1 bis 3 Ringglieder gleiche oder verschiedene Heteroatome sind. Als Heteroatome bzw. Heterogruppierungen seien genannt: Stickstoff, Sauerstoff, Schwefel, Sulfinyl und Sulfonyl, bevorzugt Stickstoff, Sauerstoff, Schwefel und Sulfonyl. Die Heterocyclen können auch benzanneliert sein. Beispielhaft seien genannt: Pyrrol, Oxazol, Imidazol, Thiazol, Pyrazol, Pyridin, Pyrimidin, Triazin, Pyridazin, Oxolan, Thiolan, Thiophen, Indol, Benzofuran, Benzoxazol und Benzimidazol, besonders bevorzugt Pyrimidin und Triazin.

Aryl, Heterocyclyl und Aralkyl in den allgemeinen Formeln können gleich oder verschieden ein- bis mehrfach, bevorzugt ein- bis fünffach, besonders bevorzugt einbis dreifach in den Ringen substituiert sein.

Als Substituenten in den Arylresten als solche als auch in den Zusammensetzungen, wie Aralkyl, seien genannt: Halogen, wie Fluor, Chlor, Brom und Jod, ganz besonders bevorzugt Chlor; Cyano, Nitro, Hydroxy, geradkettiges oder verzweigtes Alkyl, Alkoxy, Dioxyalkylen, Alkylcarbonyloxy und Alkylthio bevorzugt mit bis zu 4 Kohlenstoffatomen je Rest, besonders bevorzugt mit 1 oder 2 Kohlenstoffatomen, geradkettiges oder verzweigtes Halogenalkyl, Halogenalkoxy und Halogenalkylthio bevorzugt mit 1 bis 4 Kohlenstoffatomen und 1 bis 3 gleichen oder verschiedenen Halogenatomen wie Fluor, Chlor, Brom und Jod, besonders bevorzugt mit 1 oder 2 Kohlenstoffatomen und 1 bis 3 gleichen oder verschiedenen Halogenatomen wie Fluor und Chlor, ferner Phenyl.

Als Substituenten für die Het-Reste in den allgemeinen Formeln seien genannt: Hydroxy, Halogen, bevorzugt Fluor, Chlor, Brom und Jod, besonders bevorzugt Fluor, Chlor und Brom, Cyano, Nitro, Amino, geradkettiges oder verzweigtes Alkyl, Alkoxy und Alkylthio bevorzugt mit je 1 bis 4 Kohlenstoffatomen, besonders bevorzugt Alkyl mit 1 bis 4, Alkoxy und Alkylthio mit 1 oder 2 Kohlenstoffatomen, geradkettiges oder verzweigtes Halogenalkyl, Halogenalkoxy und Halogenalkylthio bevorzugt mit je 1 bis 4 Kohlenstoffatomen und 1 bis 9 gleichen oder verschiedenen Halogenatomen wie Fluor, Chlor, Brom und Jod, besonders bevorzugt mit 1 oder 2 Kohlenstoffatomen und 1 bis 3 gleichen oder verschiedenen Halogenatomen wie Fluor und Chlor, geradkettiges oder verzweigtes Alkylamino, Dialkylamino, Alkoxycarbonyl und Alkoximinoalkyl bevorzugt mit 1 bis 4 Kohlenstoffatomen je Alk-Rest, besonders bevorzugt mit 1 oder 2 Kohlenstoffatomen je Alk-Rest, ferner Phenyl oder bevorzugt ein- bis fünffach, besonders bevorzugt ein- bis dreifach, gleich oder verschieden durch Nitro, Halogen, wie Fluor, Chlor, Brom und Jod, besonders bevorzugt Chlor, geradkettiges oder verzweigtes Alkyl oder Alkoxy mit bevorzugt 1 bis 4, besonders bevorzugt 1 oder 2 Kohlenstoffatomen substituiertes Phenyl.

Die erfindungsgemäßen substituierten 4-Heterocyclyloximino-pyrazolin-5-one sind durch die Formel (I) allgemein definiert. Bevorzugt sind Verbindungen der Formel (I), bei welchen

$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für unsubstituiertes oder einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil oder für unsubstituiertes oder einfach bis mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder Phenyl und

Het für einen unsubstituierten oder einfach bis mehrfach, gleich oder verschieden substituierten und/oder benzannellierten, gesättigten oder ungesättigten fünfgliedrigen oder sechsgliedrigen Heterocyclus mit 1 bis 3 gleichen oder verschiedenen Heteroatomen bzw. Heterogruppierungen, insbesondere Stickstoff, Sauerstoff, Schwefel, Sulfinyl oder Sulfonyl steht, wobei als Substituenten infrage kommen: Hydroxy, Halogen, Cyano, Nitro, Amino, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkylamino, Dialkylamino, Alkoxycarbonyl oder Alkoximinoalkyl mit jeweils 1 bis 4 Kohlenstoffatomen in den einzelnen Alkylteilen oder unsubstituiertes oder einfach bis mehrfach, gleich oder verschieden durch Nitro, Halogen und/oder jeweils geradkettiges oder verzweigtes Alkyl oder Alkoxy mit jeweils mit 1 bis 4 Kohlenstoffatomen substituiertes Phenyl.

Besonders bevorzugt sind Verbindungen der Formel (I), bei welchen

$R^1$ und $R^2$ unabhängig voneinander jeweils für Wasserstoff, Methyl, Ethyl, n- oder i-Propyl, n-, i-, s- oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl, Oxiranylmethyl, Oxiranylethyl, für 1,1-Dioxotetrahydrothien-3-yl oder für unsubstituiertes oder jeweils ein- bis dreifach, gleich oder verschieden substituiertes Phenyl oder Benzyl stehen, wobei als Phenyl-Substituenten jeweils in Frage kommen: Fluor, Chlor, Brom, Iod, Cyano, Nitro, Hydroxy, Methyl, Ethyl, n- oder i-Propyl, n-, i-, s- oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und

Het für einen unsubstituierten oder ein- bis dreifach, gleich oder verschieden substituierten und/oder benzannellierten über ein Kohlenstoffatom verknüpften Heterocyclus der Formel

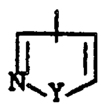 ; 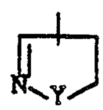 ; 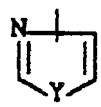 ; 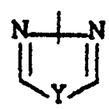 ;

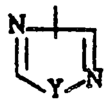 ; 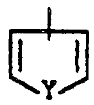 ; 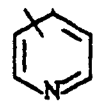 ; 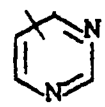 ;

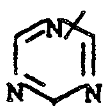 oder 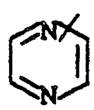 steht,

wobei als Substitutenten jeweils in Frage kommen: Hydroxy, Amino, Cyano, Nitro, Fluor, Chlor, Brom, Methyl, Ethyl, n- oder i-Propyl, n-, i-, s- oder t-Butyl, Methoxy, Ethoxy, Methylthio, Ethylthio, Trifluormethyl, Trichlormethyl, Dichlormethyl, Chlormethyl, Difluormethyl, Fluormethyl, Trifluormethoxy, Difluormethoxy, Trifluormethylthio, Methylamino, Ethylamino, Dimethylamino, Diethylamino, Methoxycarbonyl, Ethoxycarbonyl, Methoximinomethyl, Ethoximinomethyl, Methox iminoethyl, Ethoximinoethyl oder unsubstituiertes oder ein- bis dreifach, gleich oder verschieden durch Chlor, Nitro, Methyl, Ethyl und/oder Methoxy substituiertes Phenyl und wobei

Y jeweils für Sauerstoff, Schwefel, Sulfinyl oder Sulfonyl steht.

Ganz besonders bevorzugt sind Verbindungen der Formel (I), bei welcher

$R^1$ und $R^2$ unabhängig voneinander jeweils für Wasserstoff, Methyl, Ethyl, n- oder i-Propyl, für Methoxymethyl, für Hydroxyethyl, für Methoxycarbonyl oder Ethoxycarbonyl oder für unsubstituiertes oder einfach oder zweifach, gleich oder verschieden durch Chlor, Nitro, Methyl, Ethyl und/oder Methoxy substituiertes Phenyl stehen und

Het für einen unsubstituierten oder ein- bis dreifach, gleich oder verschieden substituierten Heterocyclus der Formel

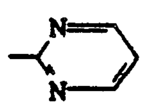 ; 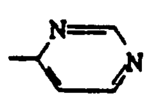 oder 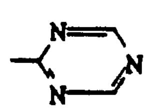 steht,

wobei als Substituenten jeweils infrage kommen: Hydroxy, Amino, Cyano, Nitro, Fluor, Chlor, Brom, Methyl, Ethyl, Methoxy, Ethoxy, Methylthio, Ethylthio, Trifluormethyl, Trichlormethyl, Di chlormethyl, Chlormethyl, Methylamino, Ethylamino, Dimethylamino, Diethylamino oder unsubstituiertes oder einfach oder zweifach, gleich oder verschieden durch Chlor, Nitro, Methyl, Ethyl und/oder Methoxy substituiertes Phenyl.

Im einzelnen seien außer den bei den Herstellungsbeispielen genannten Verbindungen die folgenden substituierten 4-Heterocyclyloximino-pyrazolin-5-one der allgemeinen Formel (I) genannt:

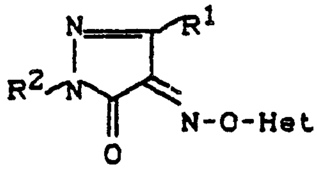

(I)

| $R^1$ | $R^2$ | Het |
|---|---|---|
| H | H | 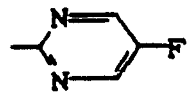 |
| H | $CH_3$ | 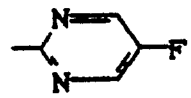 |
| H | 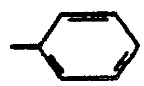 | 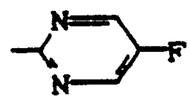 |
| H | 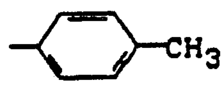 | 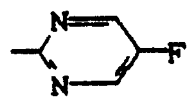 |
| $CH_3$ | H | 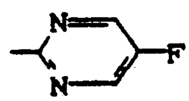 |
| $CH_3$ | $CH_3$ | 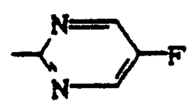 |
| $CH_3$ | 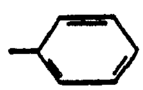 | 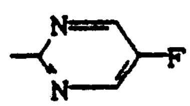 |
| $CH_3$ | 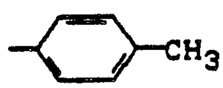 | 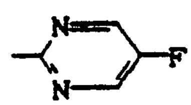 |
| $CH_3O-CH_2-$ | H | 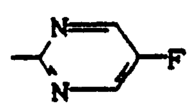 |
| $CH_3O-CH_2-$ | $CH_3$ | 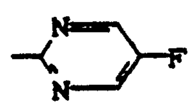 |
| $CH_3O-CH_2-$ | 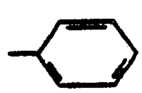 | 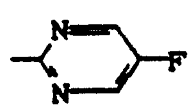 |

| $R^1$ | $R^2$ | Het |
|---|---|---|
| $CH_3O-CH_2-$ | 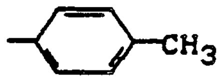 | 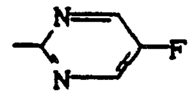 |
| 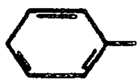 | H | 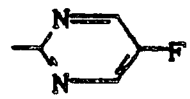 |
| 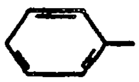 | $CH_3$ | 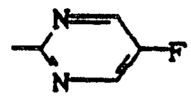 |
| 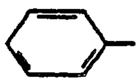 | 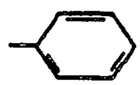 | 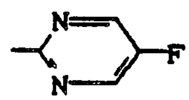 |
| 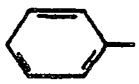 | 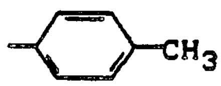 | 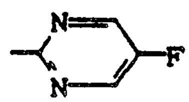 |
| H | H | 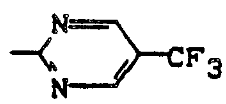 |
| H | $CH_3$ | 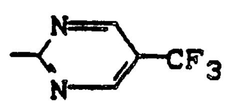 |
| H | 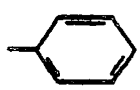 | 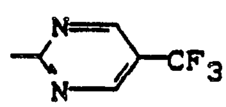 |
| H | 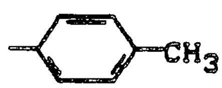 | 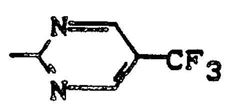 |
| $CH_3$ | H | 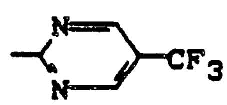 |
| $CH_3$ | $CH_3$ | 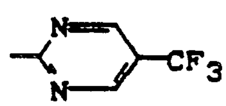 |

| $R^1$ | $R^2$ | Het |
|---|---|---|
| $CH_3$ | 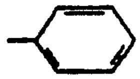 | 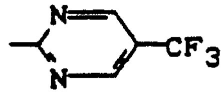 |
| $CH_3$ | 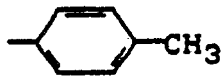 | 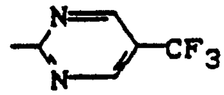 |
| $CH_3O-CH_2-$ | H | 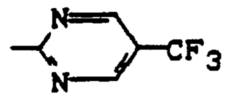 |
| $CH_3O-CH_2-$ | H | 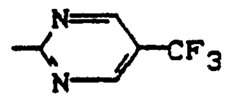 |
| $CH_3O-CH_2-$ | $CH_3$ | 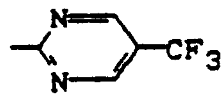 |
| $CH_3O-CH_2-$ | 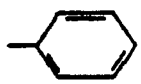 | 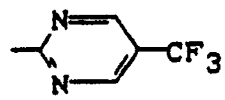 |
| $CH_3O-CH_2-$ | 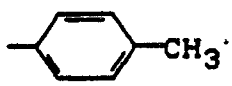 | 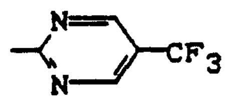 |
| 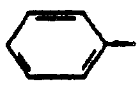 | H | 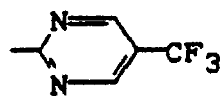 |
| 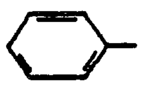 | $CH_3$ | 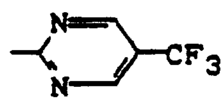 |
| 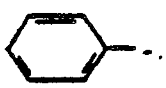 | 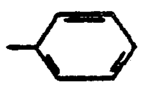 | 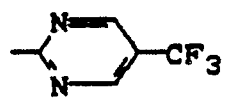 |
| 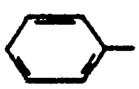 | 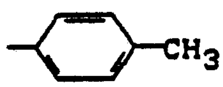 | 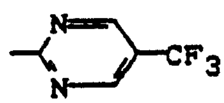 |

| $R^1$ | $R^2$ | Het |
|---|---|---|
| H | H | 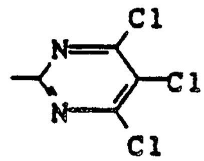 |
| H | $CH_3$ | 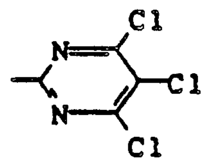 |
| H | 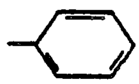 | 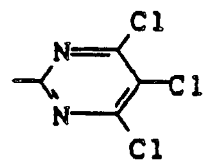 |
| H | 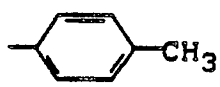 | 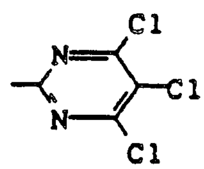 |
| $CH_3$ | H | 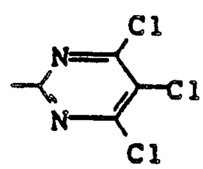 |
| $CH_3$ | $CH_3$ | 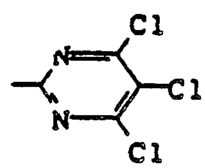 |
| $CH_3$ | 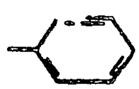 | 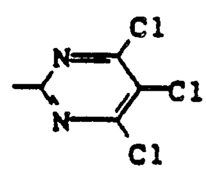 |

| $R^1$ | $R^2$ | Het |
|---|---|---|
| $CH_3$ | 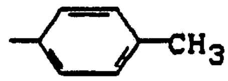 | 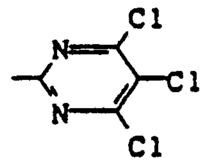 |
| $CH_3O-CH_2-$ | H | 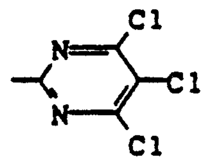 |
| $CH_3O-CH_2-$ | $CH_3$ | 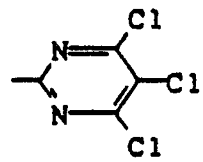 |
| $CH_3O-CH_2-$ | 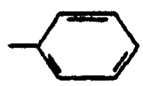 | 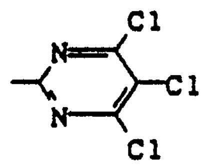 |
| $CH_3O-CH_2-$ | 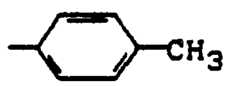 | 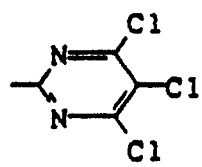 |
| 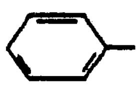 | H | 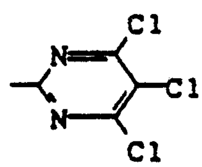 |
| 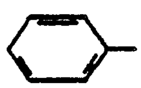 | $CH_3$ | 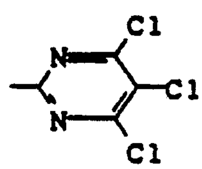 |

| $R^1$ | $R^2$ | Het |
|---|---|---|
| 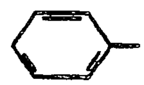 | 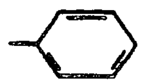 | 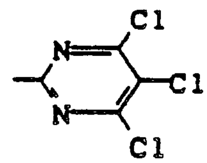 |
| 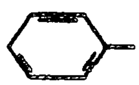 | 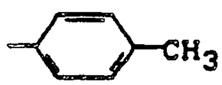 | 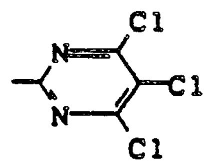 |
| H | H | 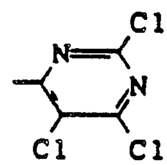 |
| H | $CH_3$ | 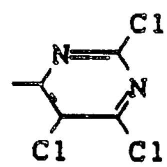 |
| H | 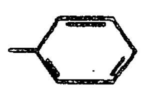 | 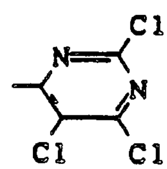 |
| H | 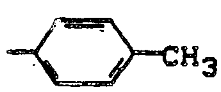 | 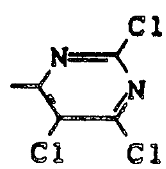 |
| $CH_3$ | H | 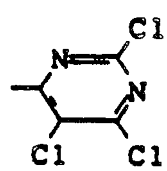 |

| $R^1$ | $R^2$ | Het |
|---|---|---|
| $CH_3$ | $CH_3$ | 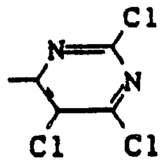 |
| $CH_3$ | 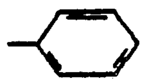 | 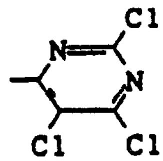 |
| $CH_3$ | 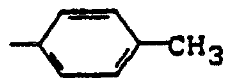 | 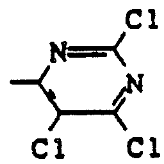 |
| $CH_3O-CH_2-$ | H | 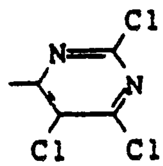 |
| $CH_3O-CH_2-$ | $CH_3$ | 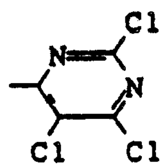 |
| $CH_3O-CH_2-$ | 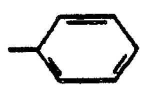 | 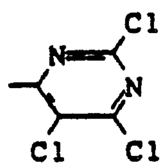 |
| $CH_3O-CH_2-$ | 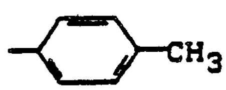 | 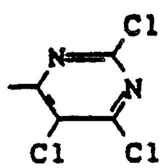 |

| $R^1$ | $R^2$ | Het |
| --- | --- | --- |
| 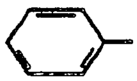 | H | 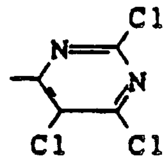 |
| 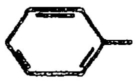 | $CH_3$ | 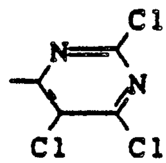 |
| 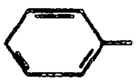 | 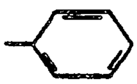 | 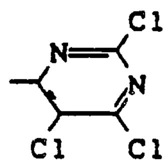 |
| 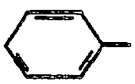 | 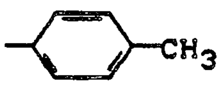 | 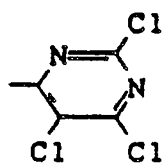 |
| H | H | 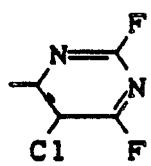 |
| H | $CH_3$ | 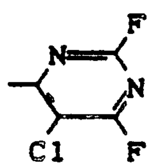 |
| H | 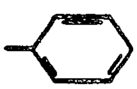 | 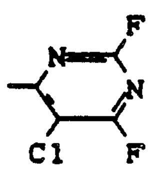 |

| $R^1$ | $R^2$ | Het |
|---|---|---|
| H | 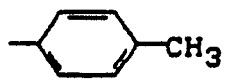 | 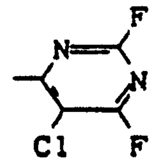 |
| $CH_3$ | H | 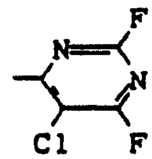 |
| $CH_3$ | $CH_3$ | 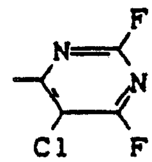 |
| $CH_3$ | 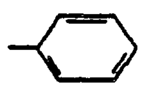 | 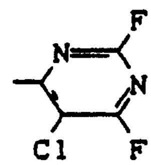 |
| $CH_3$ | 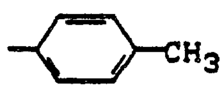 | 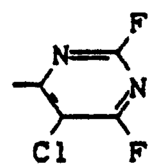 |
| $CH_3O-CH_2-$ | H | 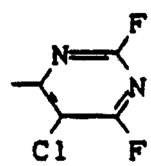 |
| $CH_3O-CH_2-$ | $CH_3$ | 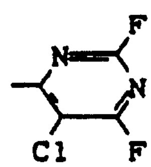 |

| $R^1$ | $R^2$ | Het |
|---|---|---|
| $CH_3O-CH_2-$ | 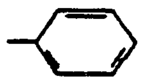 | 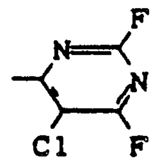 |
| $CH_3O-CH_2-$ | 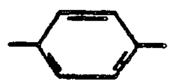 | 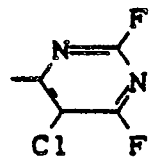 |
| 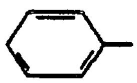 | H | 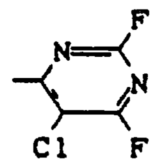 |
| 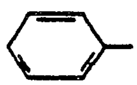 | $CH_3$ | 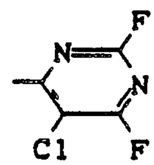 |
| 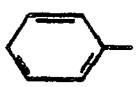 | 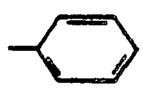 | 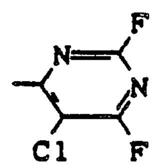 |
| 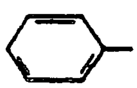 | 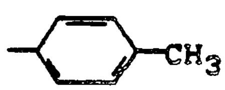 | 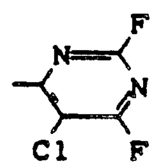 |
| H | H | 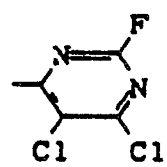 |

| $R^1$ | $R^2$ | Het |
|---|---|---|
| H | $CH_3$ | 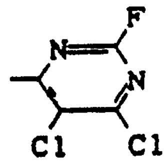 |
| H | 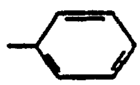 | 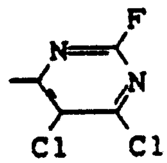 |
| H | 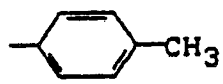 | 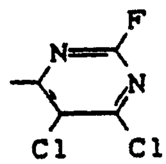 |
| $CH_3$ | H | 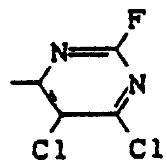 |
| $CH_3$ | $CH_3$ | 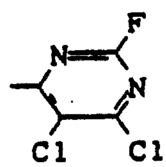 |
| $CH_3$ | 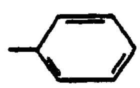 | 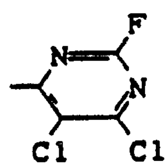 |
| $CH_3$ | 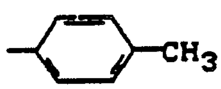 | 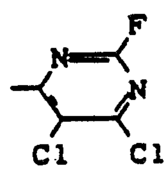 |

| $R^1$ | $R^2$ | Het |
|---|---|---|
| $CH_3O-CH_2-$ | H | 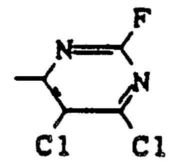 |
| $CH_3O-CH_2-$ | $CH_3$ | 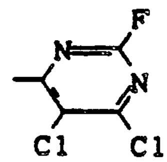 |
| $CH_3O-CH_2-$ | 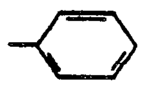 | 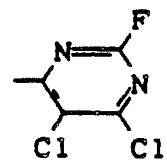 |
| $CH_3O-CH_2-$ | 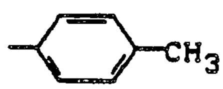 | 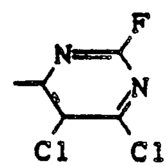 |
| 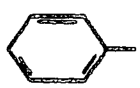 | H | 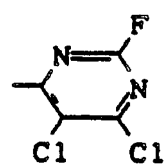 |
| 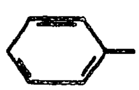 | $CH_3$ | 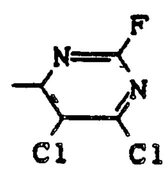 |
| 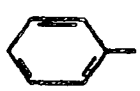 | 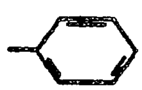 | 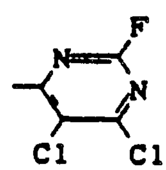 |

| $R^1$ | $R^2$ | Het |
|---|---|---|
| 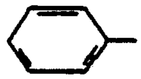 | 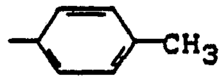 | 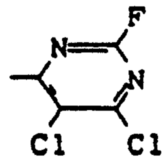 |
| H | H | 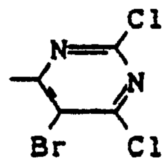 |
| H | $CH_3$ | 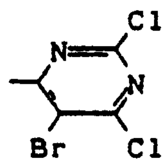 |
| H | 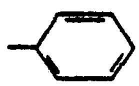 | 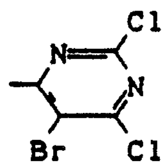 |
| H | 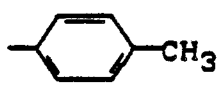 | 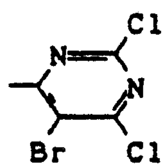 |
| $CH_3$ | H | 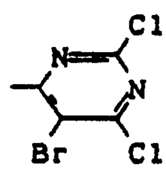 |
| $CH_3$ | $CH_3$ | 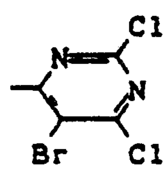 |

| $R^1$ | $R^2$ | Het |
|---|---|---|
| $CH_3$ | 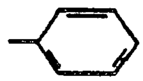 | 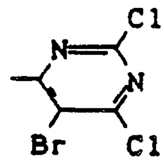 |
| $CH_3$ | 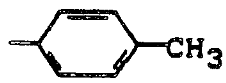 | 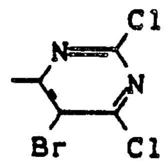 |
| $CH_3O-CH_2-$ | H | 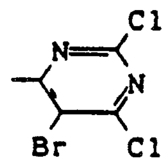 |
| $CH_3O-CH_2-$ | $CH_3$ | 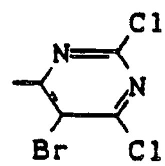 |
| $CH_3O-CH_2-$ | 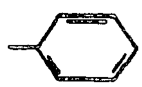 | 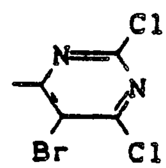 |
| $CH_3O-CH_2-$ | 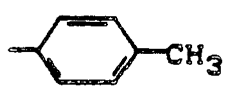 | 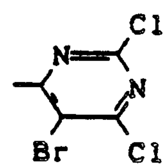 |
| 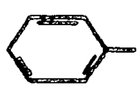 | H | 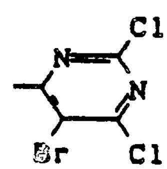 |

| $R^1$ | $R^2$ | Het |
|---|---|---|
| 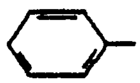 | $CH_3$ | 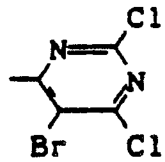 |
| 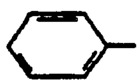 | 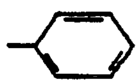 | 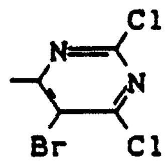 |
| 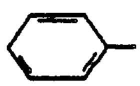 | 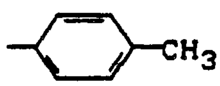 | 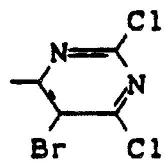 |
| H | H | 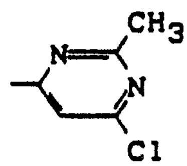 |
| H | $CH_3$ | 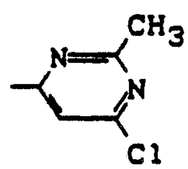 |
| H | 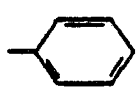 | 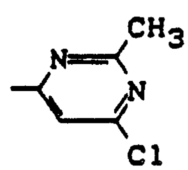 |
| H | 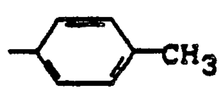 | 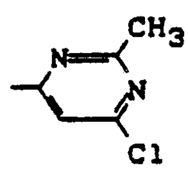 |

| $R^1$ | $R^2$ | Het |
|---|---|---|
| $CH_3$ | H | 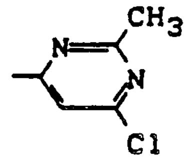 |
| $CH_3$ | $CH_3$ | 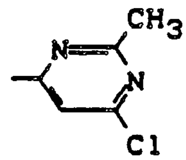 |
| $CH_3$ | 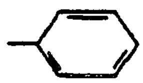 | 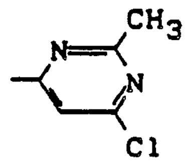 |
| $CH_3$ | 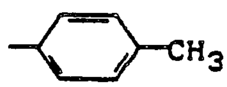 | 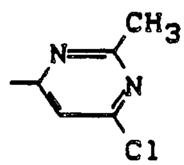 |
| $CH_3O-CH_2-$ | H | 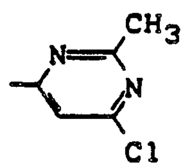 |
| $CH_3O-CH_2-$ | $CH_3$ | 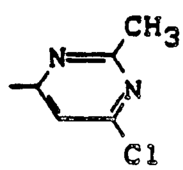 |
| $CH_3O-CH_2-$ | 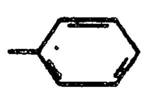 | 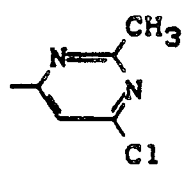 |

| $R^1$ | $R^2$ | Het |
|---|---|---|
| $CH_3O-CH_2-$ | 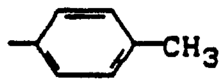 | 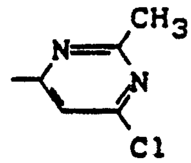 |
| 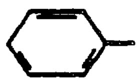 | H | 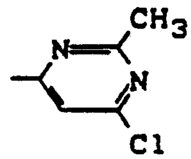 |
| 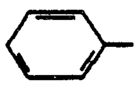 | $CH_3$ | 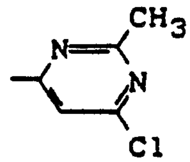 |
| 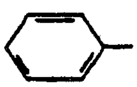 | 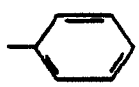 | 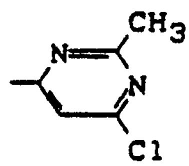 |
| 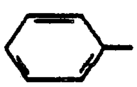 | 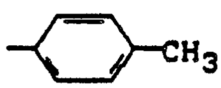 | 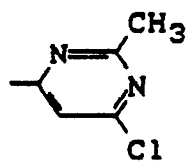 |
| H | H | 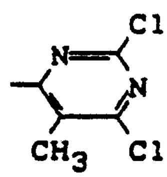 |
| H | $CH_3$ | 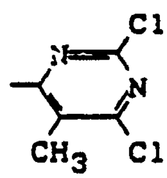 |

| $R^1$ | $R^2$ | Het |
|---|---|---|
| H | 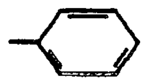 | 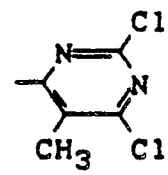 |
| H | 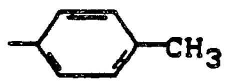 | 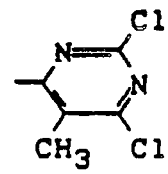 |
| $CH_3$ | H | 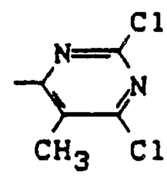 |
| $CH_3$ | $CH_3$ | 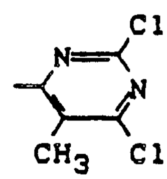 |
| $CH_3$ | 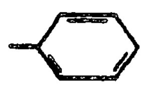 | 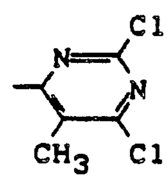 |
| $CH_3$ | 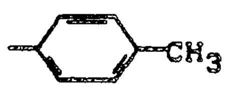 | 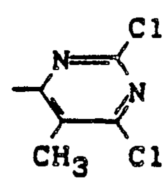 |
| $CH_3O-CH_2-$ | H | 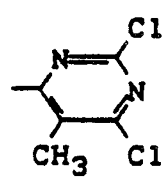 |

| $R^1$ | $R^2$ | Het |
|---|---|---|
| $CH_3O-CH_2-$ | $CH_3$ | 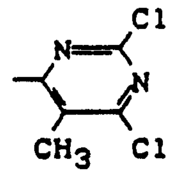 |
| $CH_3O-CH_2-$ | 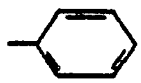 | 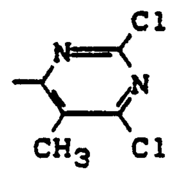 |
| $CH_3O-CH_2-$ | 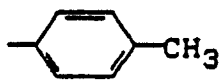 | 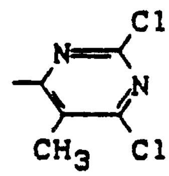 |
| 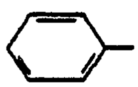 | H | 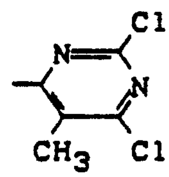 |
| 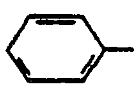 | $CH_3$ | 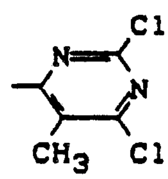 |
| 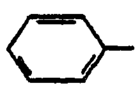 | 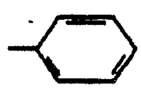 | 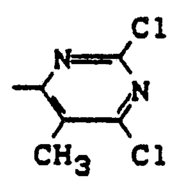 |
| 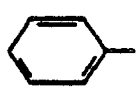 | 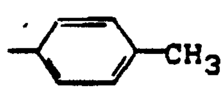 | 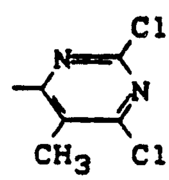 |

| $R^1$ | $R^2$ | Het |
|---|---|---|
| H | H | 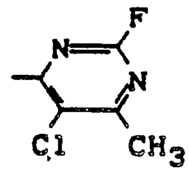 |
| H | $CH_3$ | 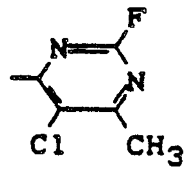 |
| H | 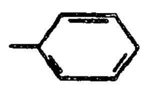 | 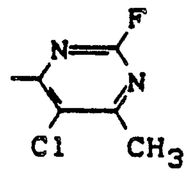 |
| H | 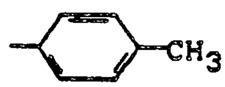 | 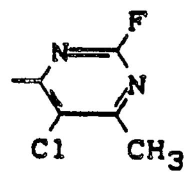 |
| $CH_3$ | H | 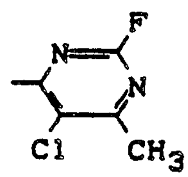 |
| $CH_3$ | $CH_3$ | 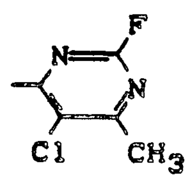 |
| $CH_3$ | 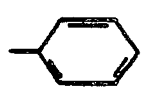 | 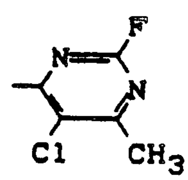 |

| $R^1$ | $R^2$ | Het |
|---|---|---|
| $CH_3$ | 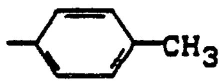 | 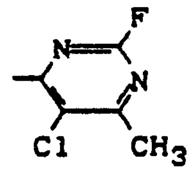 |
| $CH_3O-CH_2-$ | H | 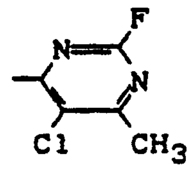 |
| $CH_3O-CH_2-$ | $CH_3$ | 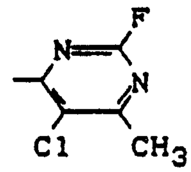 |
| $CH_3O-CH_2-$ | 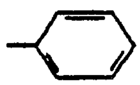 | 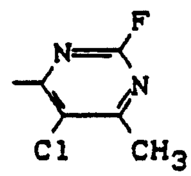 |
| $CH_3O-CH_2-$ | 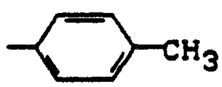 | 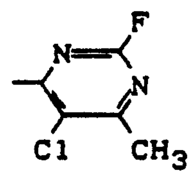 |
| 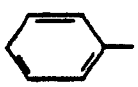 | H | 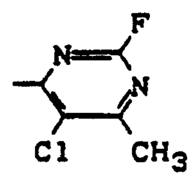 |
| 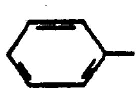 | $CH_3$ | 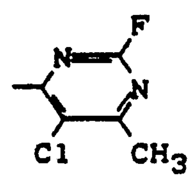 |

| $R^1$ | $R^2$ | Het |
|---|---|---|
| 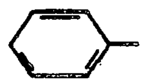 | 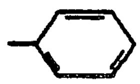 | 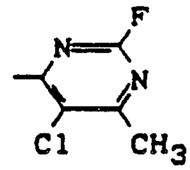 |
| 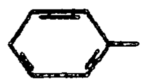 | 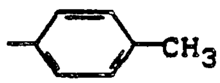 | 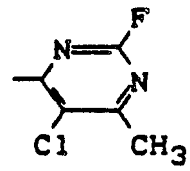 |
| H | H | 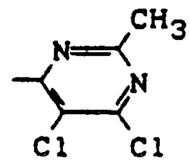 |
| H | $CH_3$ | 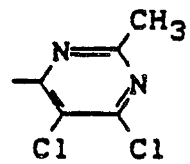 |
| H | 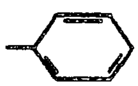 | 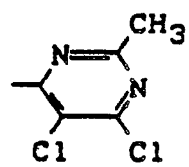 |
| H | 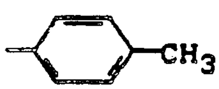 | 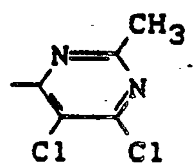 |
| $CH_3$ | H | 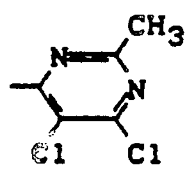 |

| $R^1$ | $R^2$ | Het |
|---|---|---|
| $CH_3$ | $CH_3$ | 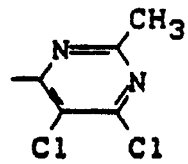 |
| $CH_3$ | 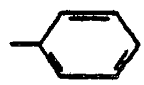 | 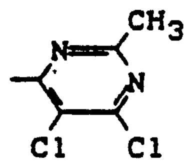 |
| $CH_3$ | 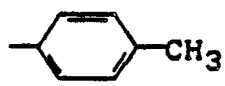 | 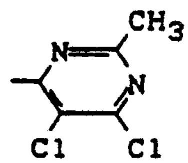 |
| $CH_3O-CH_2-$ | H | 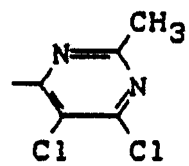 |
| $CH_3O-CH_2-$ | $CH_3$ | 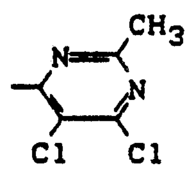 |
| $CH_3O-CH_2-$ | 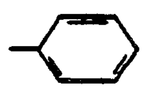 | 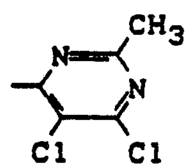 |
| $CH_3O-CH_2-$ | 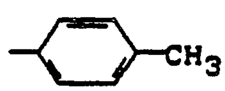 | 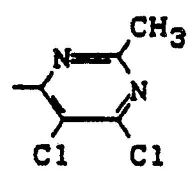 |

| $R^1$ | $R^2$ | Het |
|---|---|---|
| 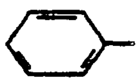 | H | 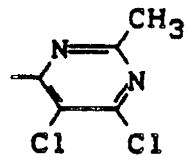 |
| 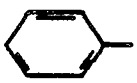 | $CH_3$ | 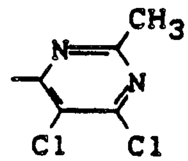 |
| 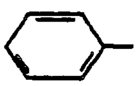 | 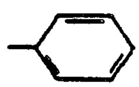 | 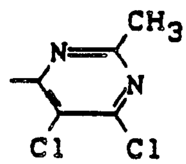 |
| 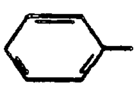 | 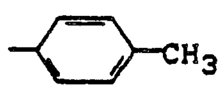 | 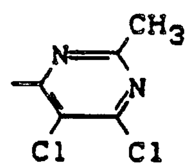 |
| H | H | 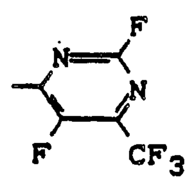 |
| H | $CH_3$ | 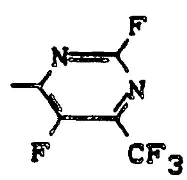 |
| H | 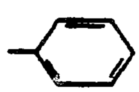 | 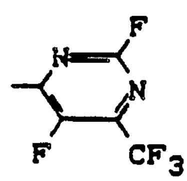 |

| $R^1$ | $R^2$ | Het |
|---|---|---|
| H | 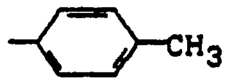 | 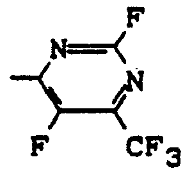 |
| $CH_3$ | H | 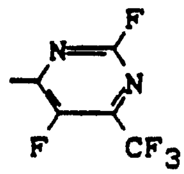 |
| $CH_3$ | $CH_3$ | 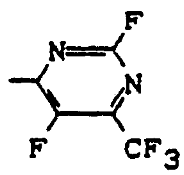 |
| $CH_3$ | 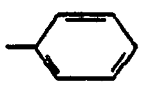 | 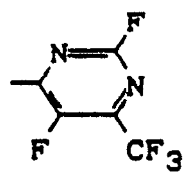 |
| $CH_3$ | 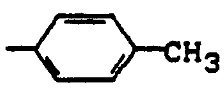 | 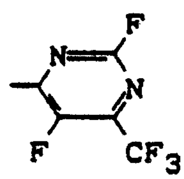 |
| $CH_3O-CH_2-$ | H | 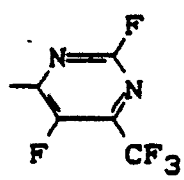 |
| $CH_3O-CH_2-$ | $CH_3$ | 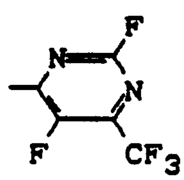 |

| $R^1$ | $R^2$ | Het |
|---|---|---|
| $CH_3O-CH_2-$ | 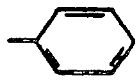 | 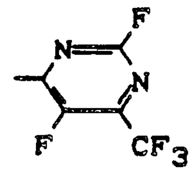 |
| $CH_3O-CH_2-$ | 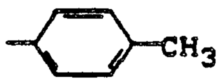 | 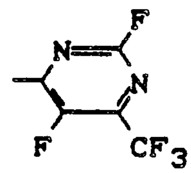 |
| 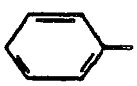 | H | 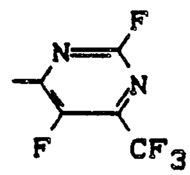 |
| 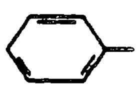 | $CH_3$ | 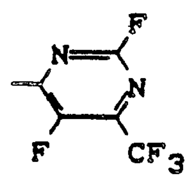 |
| 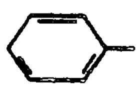 | 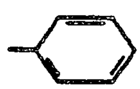 | 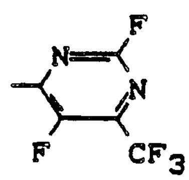 |
| 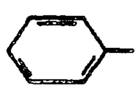 | 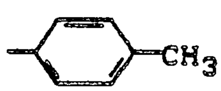 | 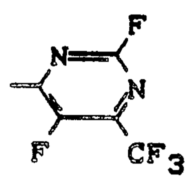 |
| H | H | 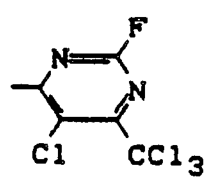 |

| $R^1$ | $R^2$ | Het |
|---|---|---|
| H | $CH_3$ | 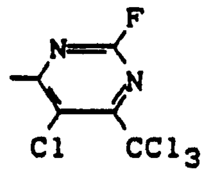 |
| H | 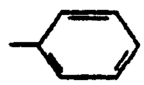 | 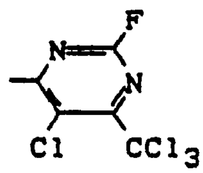 |
| H | 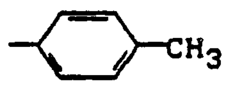 | 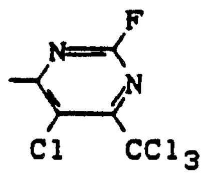 |
| $CH_3$ | H | 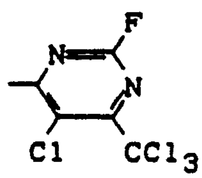 |
| $CH_3$ | $CH_3$ | 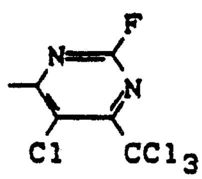 |
| $CH_3$ | 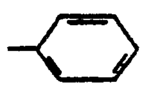 | 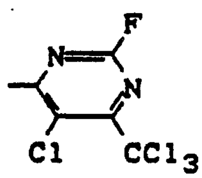 |
| $CH_3$ | 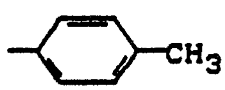 | 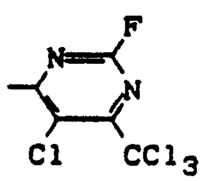 |

| $R^1$ | $R^2$ | Het |
|---|---|---|
| $CH_3O-CH_2-$ | H | 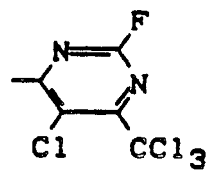 |
| $CH_3O-CH_2-$ | $CH_3$ | 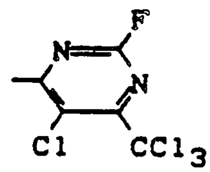 |
| $CH_3O-CH_2-$ | 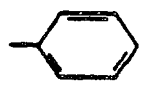 | 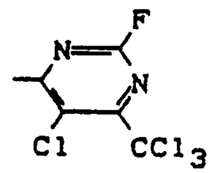 |
| $CH_3O-CH_2-$ | 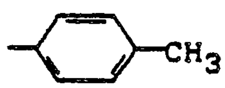 | 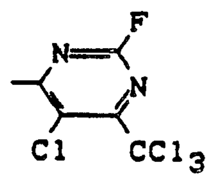 |
| 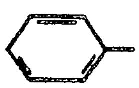 | H | 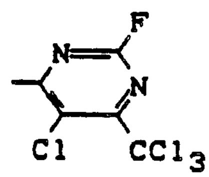 |
| 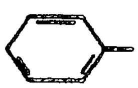 | $CH_3$ | 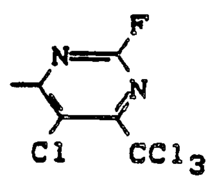 |
| 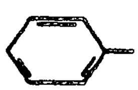 | 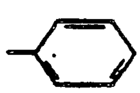 | 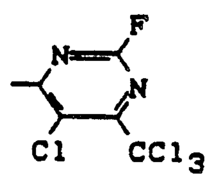 |

| $R^1$ | $R^2$ | Het |
|---|---|---|
| 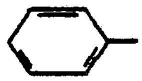 | 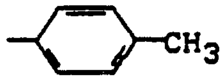 | 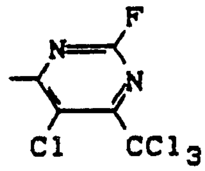 |
| H | H | 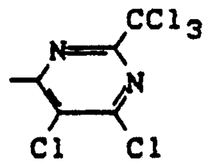 |
| H | $CH_3$ | 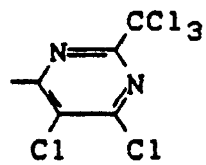 |
| H | 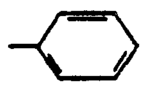 | 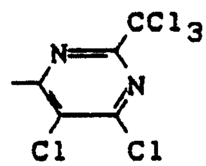 |
| H | 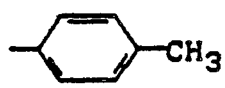 | 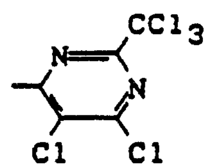 |
| $CH_3$ | H | 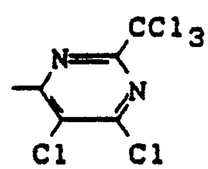 |
| $CH_3$ | $CH_3$ | 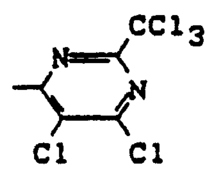 |

| $R^1$ | $R^2$ | Het |
|---|---|---|
| $CH_3$ | 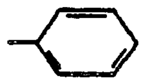 | 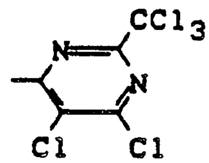 |
| $CH_3$ | 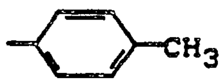 | 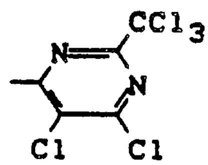 |
| $CH_3O-CH_2-$ | H | 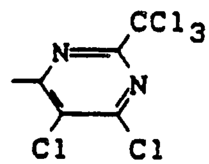 |
| $CH_3O-CH_2-$ | $CH_3$ | 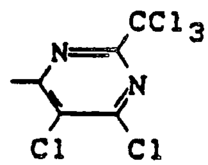 |
| $CH_3O-CH_2-$ | 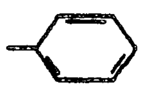 | 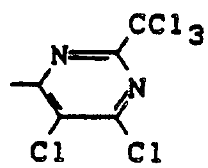 |
| $CH_3O-CH_2-$ | 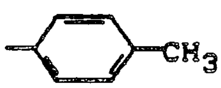 | 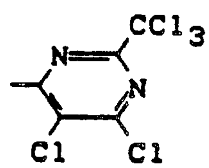 |
| 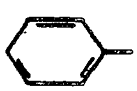 | H | 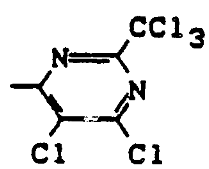 |

| $R^1$ | $R^2$ | Het |
|---|---|---|
| 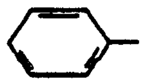 | $CH_3$ | 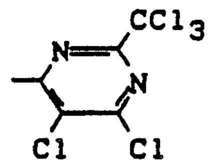 |
| 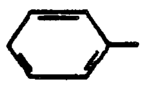 | 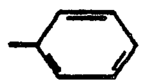 | 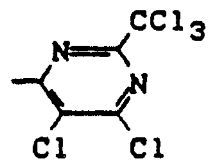 |
| 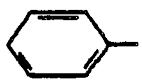 | 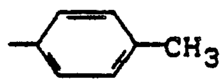 | 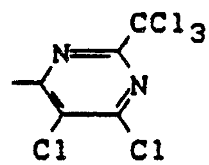 |
| H | H | 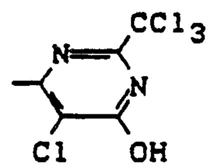 |
| H | $CH_3$ | 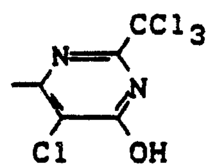 |
| H | 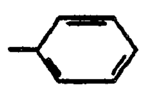 | 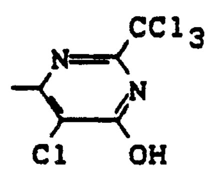 |
| H | 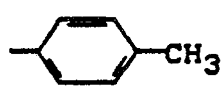 | 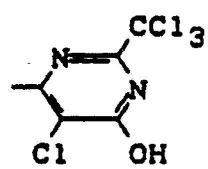 |

| $R^1$ | $R^2$ | Het |
|---|---|---|
| $CH_3$ | H | 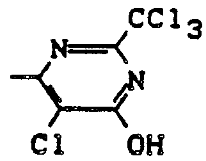 |
| $CH_3$ | $CH_3$ | 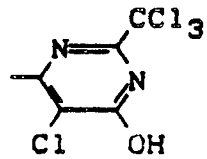 |
| $CH_3$ | 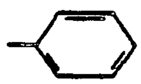 | 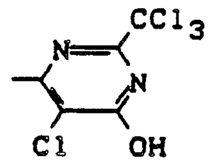 |
| $CH_3$ | 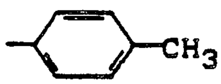 | 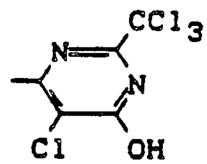 |
| $CH_3O-CH_2-$ | H | 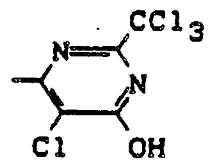 |
| $CH_3O-CH_2-$ | $CH_3$ | 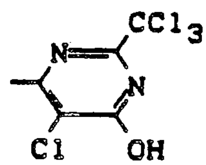 |
| $CH_3O-CH_2-$ | 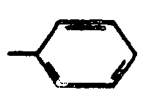 | 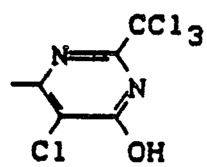 |

| $R^1$ | $R^2$ | Het |
|---|---|---|
| $CH_3O-CH_2-$ | 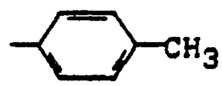 | 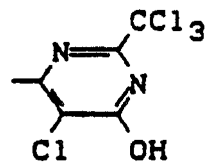 |
| 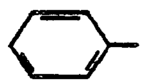 | H | 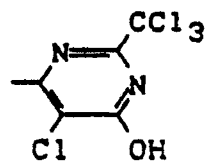 |
| 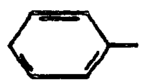 | $CH_3$ | 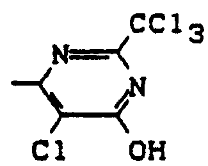 |
| 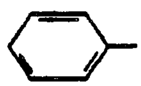 | 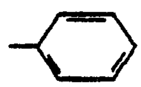 | 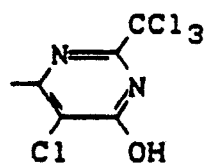 |
| 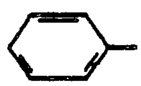 | 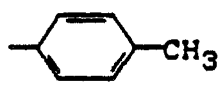 | 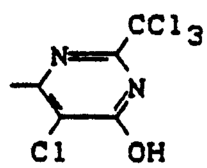 |
| H | H | 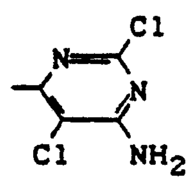 |
| H | $CH_3$ | 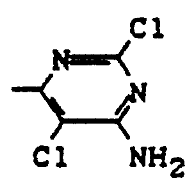 |

| $R^1$ | $R^2$ | Het |
|---|---|---|
| H | 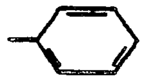 | 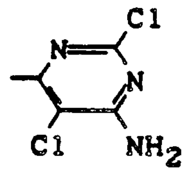 |
| H | 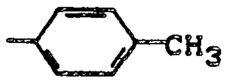 | 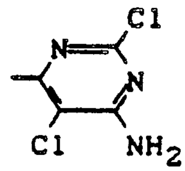 |
| $CH_3$ | H | 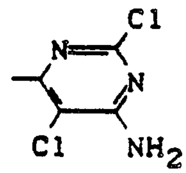 |
| $CH_3$ | $CH_3$ | 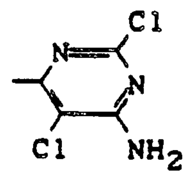 |
| $CH_3$ | 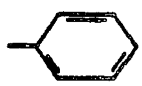 | 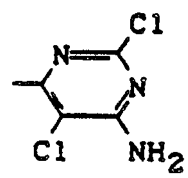 |
| $CH_3$ | 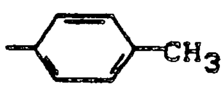 | 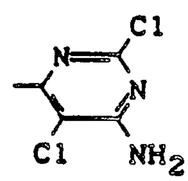 |
| $CH_3O-CH_2-$ | H | 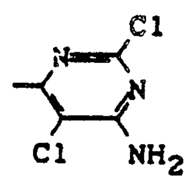 |

| $R^1$ | $R^2$ | Het |
|---|---|---|
| $CH_3O-CH_2-$ | $CH_3$ | 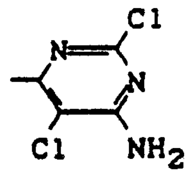 |
| $CH_3O-CH_2-$ | 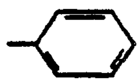 | 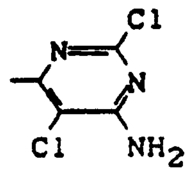 |
| $CH_3O-CH_2-$ | 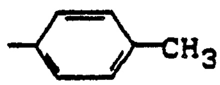 | 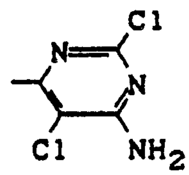 |
| 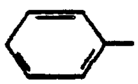 | H | 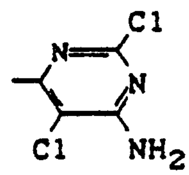 |
| 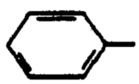 | $CH_3$ | 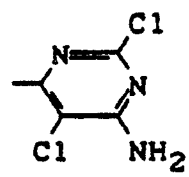 |
| 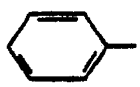 | 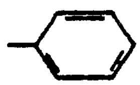 | 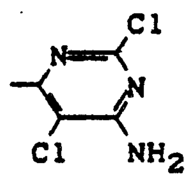 |
| 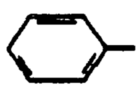 | 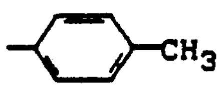 | 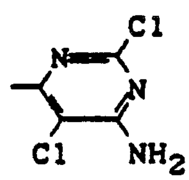 |

| $R^1$ | $R^2$ | Het |
|---|---|---|
| H | H | 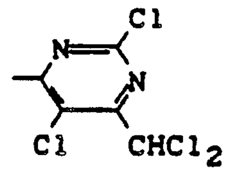 |
| H | $CH_3$ | 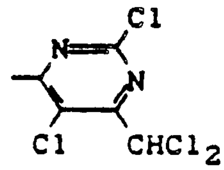 |
| H | 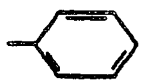 | 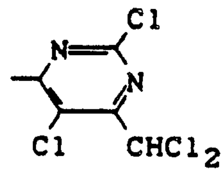 |
| H | 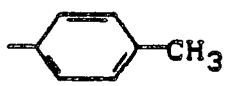 | 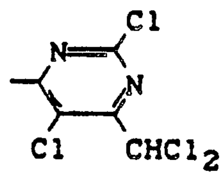 |
| $CH_3$ | H | 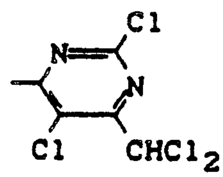 |
| $CH_3$ | $CH_3$ | 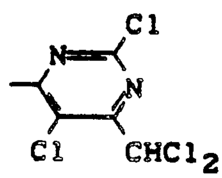 |
| $CH_3$ | 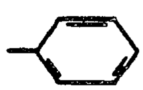 | 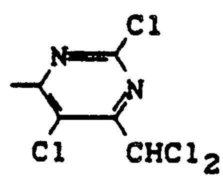 |

| $R^1$ | $R^2$ | Het |
|---|---|---|
| $CH_3$ | 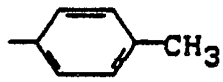 | 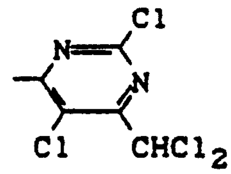 |
| $CH_3O-CH_2-$ | H | 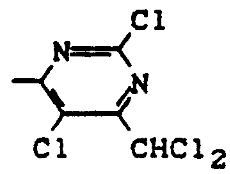 |
| $CH_3O-CH_2-$ | $CH_3$ | 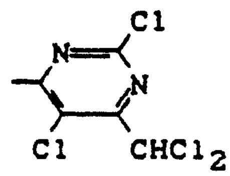 |
| $CH_3O-CH_2-$ | 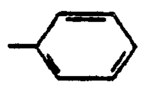 | 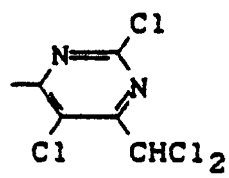 |
| $CH_3O-CH_2-$ | 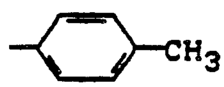 | 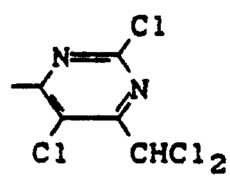 |
| 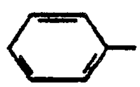 | H | 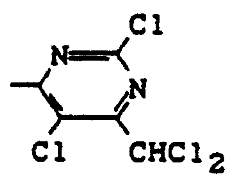 |
| 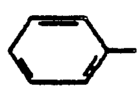 | $CH_3$ | 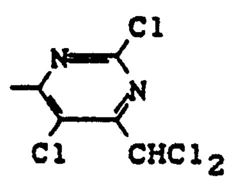 |

| $R^1$ | $R^2$ | Het |
|---|---|---|
| 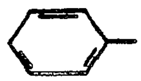 | 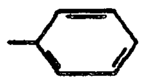 | 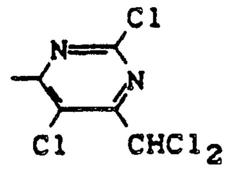 |
| 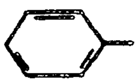 | 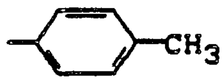 | 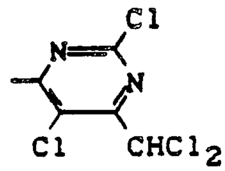 |
| H | H | 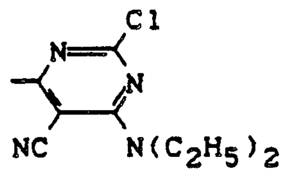 |
| H | $CH_3$ | 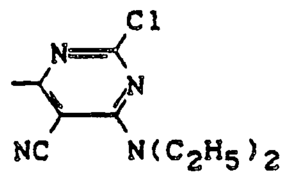 |
| H | 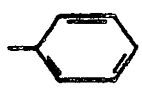 | 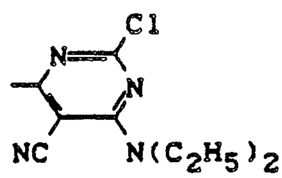 |
| H | 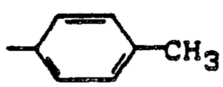 | 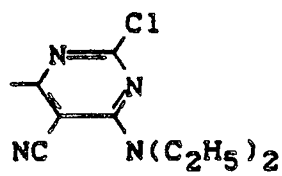 |
| $CH_3$ | H | 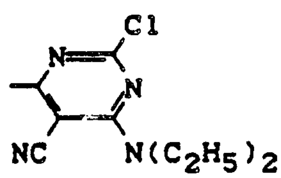 |

| $R^1$ | $R^2$ | Het |
|---|---|---|
| $CH_3$ | $CH_3$ | 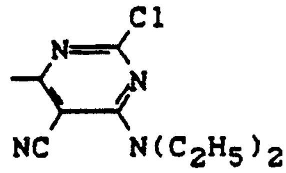 |
| $CH_3$ | 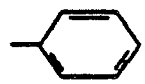 | 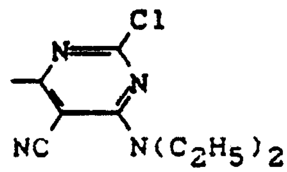 |
| $CH_3$ | 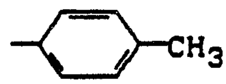 | 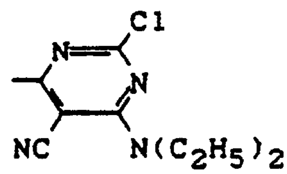 |
| $CH_3O-CH_2-$ | H | 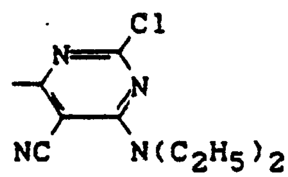 |
| $CH_3O-CH_2-$ | $CH_3$ | 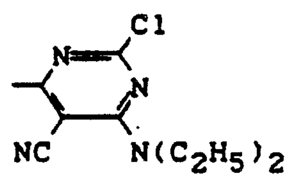 |
| $CH_3O-CH_2-$ | 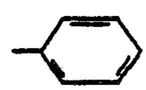 | 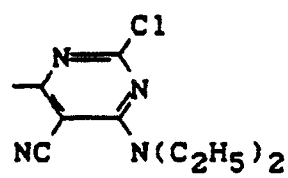 |
| $CH_3O-CH_2-$ | 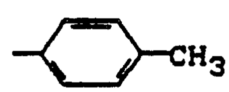 | 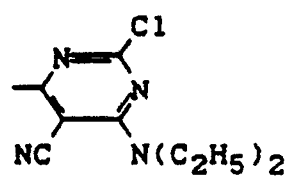 |

| $R^1$ | $R^2$ | Het |
|---|---|---|
| 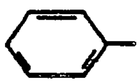 | H | 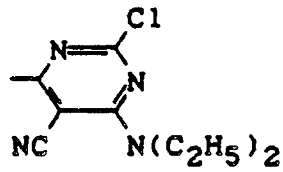 |
| 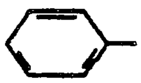 | $CH_3$ | 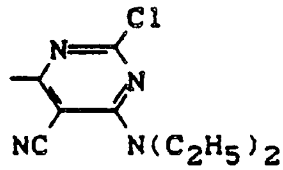 |
| 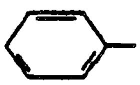 | 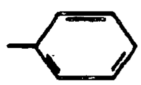 | 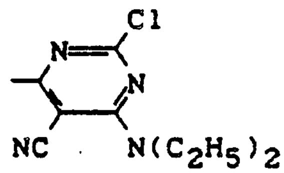 |
| 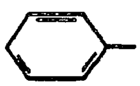 | 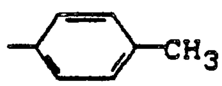 | 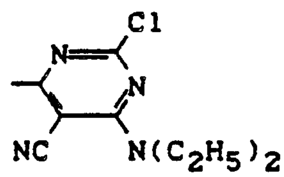 |
| H | H | 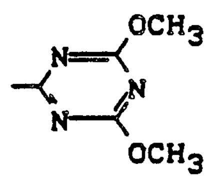 |
| H | $CH_3$ | 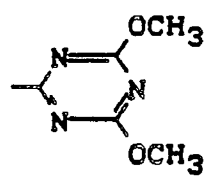 |
| H | 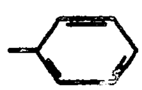 | 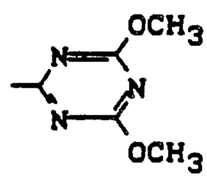 |

| $R^1$ | $R^2$ | Het |
|---|---|---|
| H | 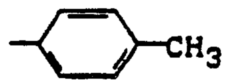 | 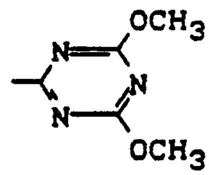 |
| $CH_3$ | H | 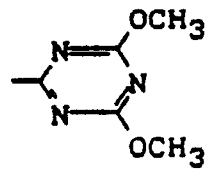 |
| $CH_3$ | $CH_3$ | 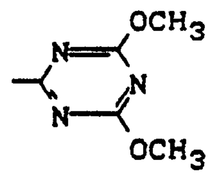 |
| $CH_3$ | 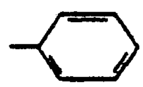 | 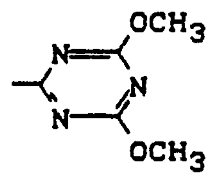 |
| $CH_3$ | 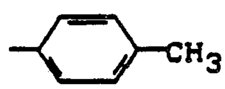 | 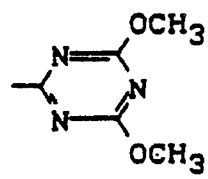 |
| $CH_3O-CH_2-$ | H | 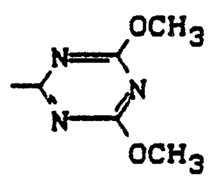 |
| $CH_3O-CH_2-$ | $CH_3$ | 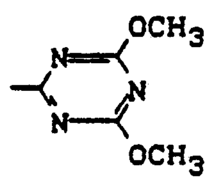 |

| $R^1$ | $R^2$ | Het |
|---|---|---|
| $CH_3O-CH_2-$ | 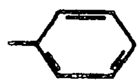 | 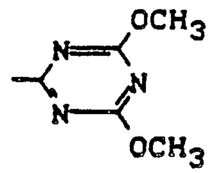 |
| $CH_3O-CH_2-$ | 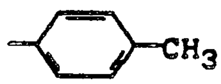 | 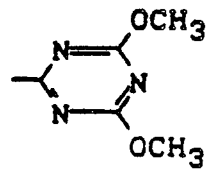 |
| 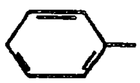 | H | 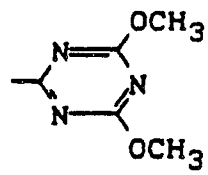 |
| 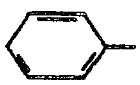 | $CH_3$ | 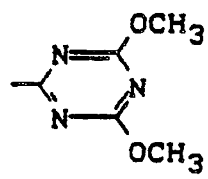 |
| 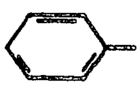 | 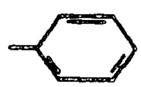 | 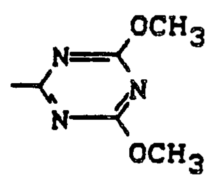 |
| 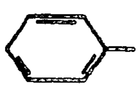 | 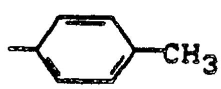 | 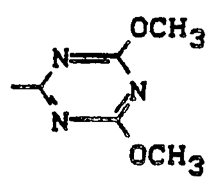 |
| H | H | 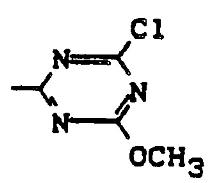 |

| $R^1$ | $R^2$ | Het |
|---|---|---|
| H | $CH_3$ | 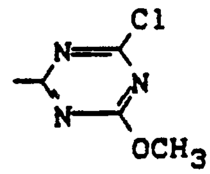 |
| H | 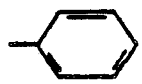 | 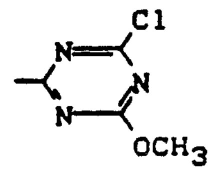 |
| H | 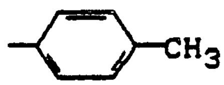 | 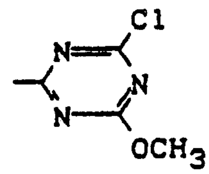 |
| $CH_3$ | H | 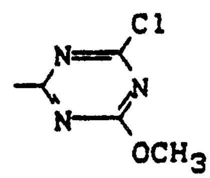 |
| $CH_3$ | $CH_3$ | 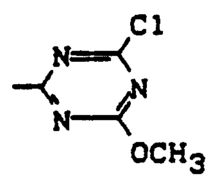 |
| $CH_3$ | 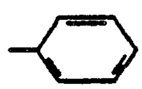 | 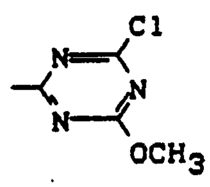 |
| $CH_3$ | 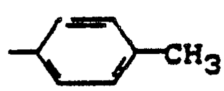 | 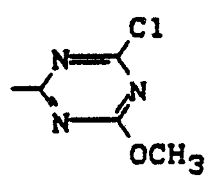 |

| $R^1$ | $R^2$ | Het |
|---|---|---|
| $CH_3O-CH_2-$ | H | 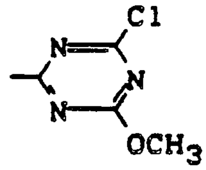 |
| $CH_3O-CH_2-$ | $CH_3$ | 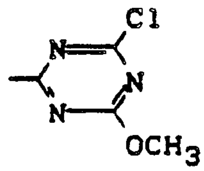 |
| $CH_3O-CH_2-$ | 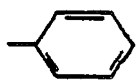 | 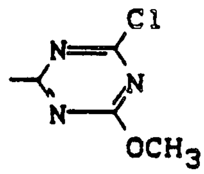 |
| $CH_3O-CH_2-$ | 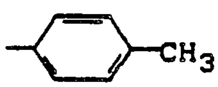 | 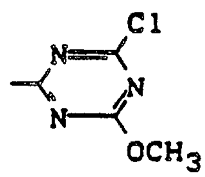 |
| 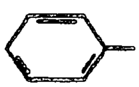 | H | 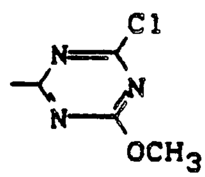 |
| 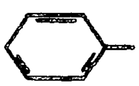 | $CH_3$ | 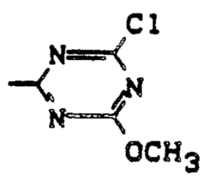 |
| 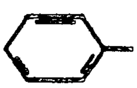 | 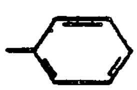 | 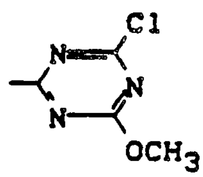 |

| $R^1$ | $R^2$ | Het |
|---|---|---|
| 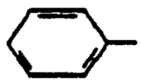 | 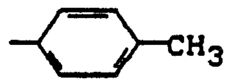 | 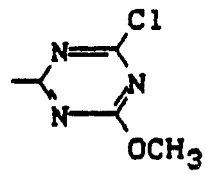 |
| H | H | 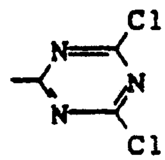 |
| H | $CH_3$ | 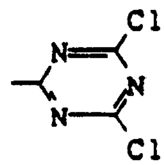 |
| H | 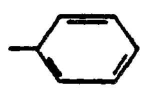 | 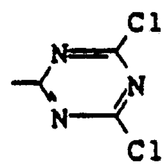 |
| H | 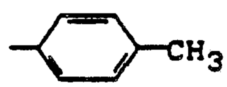 | 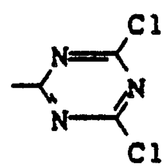 |
| $CH_3$ | H | 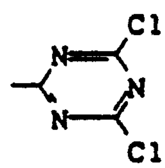 |
| $CH_3$ | $CH_3$ | 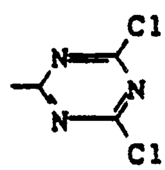 |

| $R^1$ | $R^2$ | Het |
|---|---|---|
| $CH_3$ | 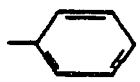 | 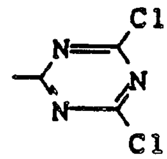 |
| $CH_3$ | 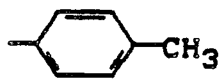 | 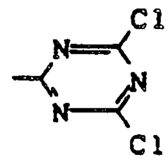 |
| $CH_3O-CH_2-$ | H | 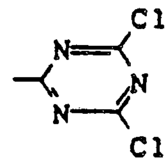 |
| $CH_3O-CH_2-$ | $CH_3$ | 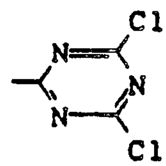 |
| $CH_3O-CH_2-$ | 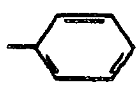 | 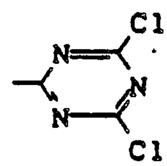 |
| $CH_3O-CH_2-$ | 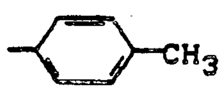 | 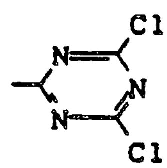 |
| 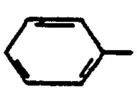 | H | 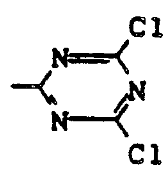 |

| $R^1$ | $R^2$ | Het |
|---|---|---|
| 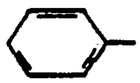 | $CH_3$ | 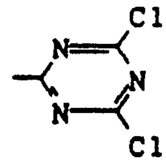 |
| 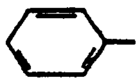 | 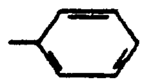 | 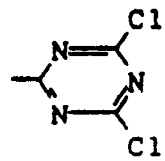 |
| 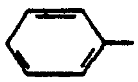 | 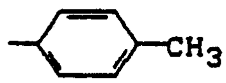 | 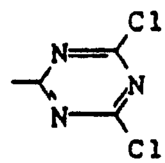 |
| H | H | 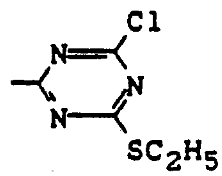 |
| H | $CH_3$ | 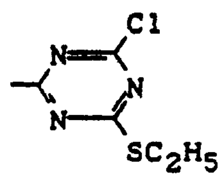 |
| H | 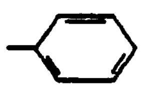 | 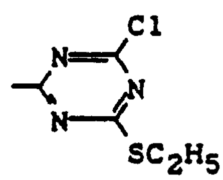 |
| H | 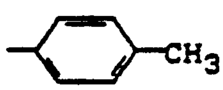 | 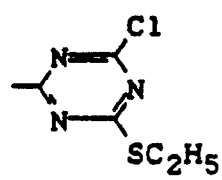 |

| $R^1$ | $R^2$ | Het |
|---|---|---|
| $CH_3$ | H | 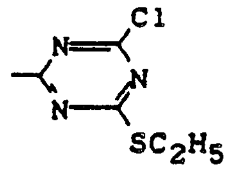 |
| $CH_3$ | $CH_3$ | 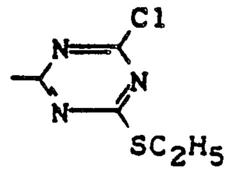 |
| $CH_3$ | 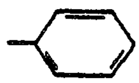 | 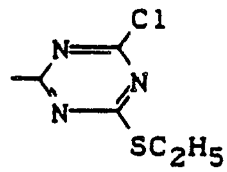 |
| $CH_3$ | 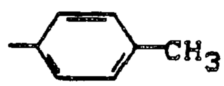 | 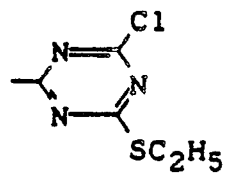 |
| $CH_3O-CH_2-$ | H | 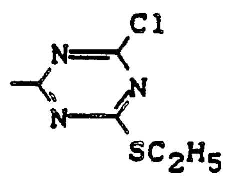 |
| $CH_3O-CH_2-$ | $CH_3$ | 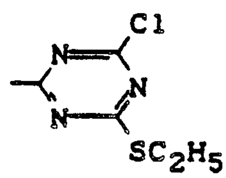 |
| $CH_3O-CH_2-$ | 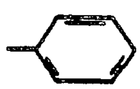 | 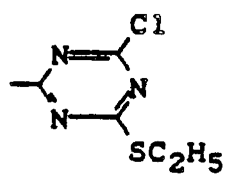 |

| $R^1$ | $R^2$ | Het |
|---|---|---|
| $CH_3O-CH_2-$ | 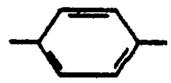 | 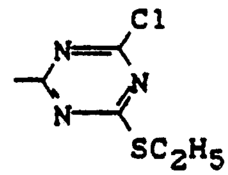 |
| 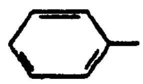 | H | 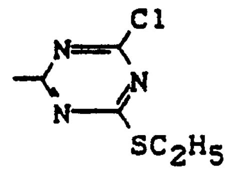 |
| 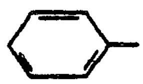 | $CH_3$ | 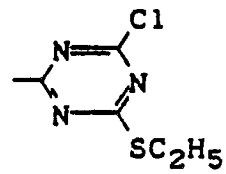 |
| 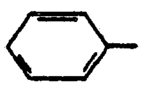 | 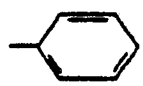 | 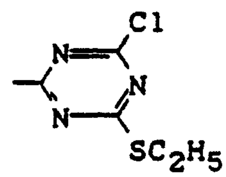 |
| 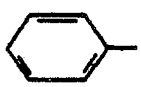 | 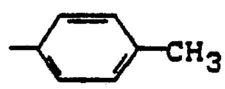 | 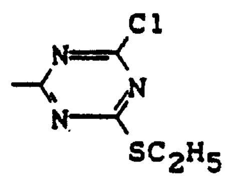 |
| H | H | 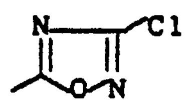 |
| H | $CH_3$ | 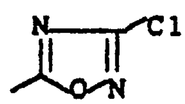 |
| H | 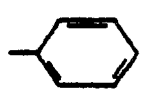 | 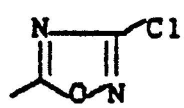 |

| $R^1$ | $R^2$ | Het |
|---|---|---|
| H | 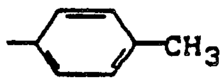 | 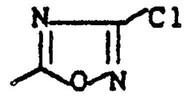 |
| $CH_3$ | H | 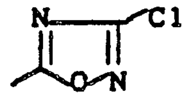 |
| $CH_3$ | $CH_3$ | 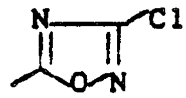 |
| $CH_3$ | 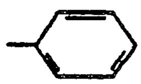 | 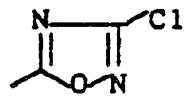 |
| $CH_3$ | 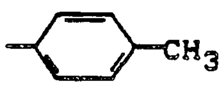 | 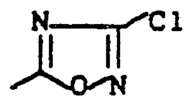 |
| $CH_3O-CH_2-$ | H | 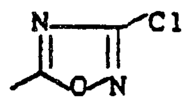 |
| $CH_3O-CH_2-$ | $CH_3$ | 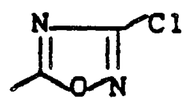 |
| $CH_3O-CH_2-$ | 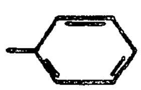 | 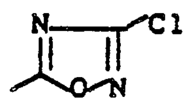 |
| $CH_3O-CH_2-$ | 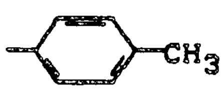 | 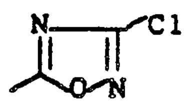 |
| 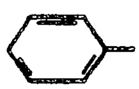 | H | 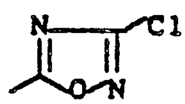 |
| 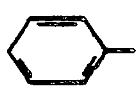 | $CH_3$ | 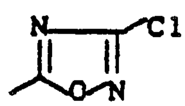 |

| $R^1$ | $R^2$ | Het |
|---|---|---|
| 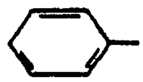 | 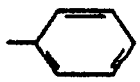 | 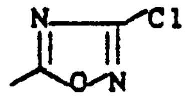 |
| 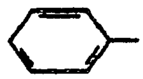 | 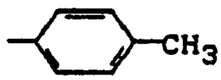 | 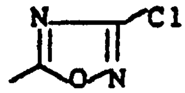 |
| H | H | 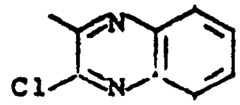 |
| H | $CH_3$ | 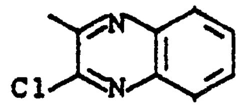 |
| H | 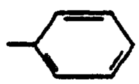 | 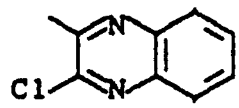 |
| H | 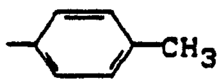 | 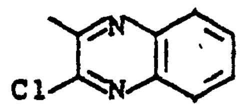 |
| $CH_3$ | H | 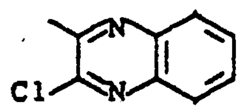 |
| $CH_3$ | $CH_3$ | 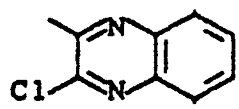 |
| $CH_3$ | 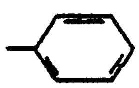 | 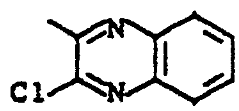 |
| $CH_3$ | 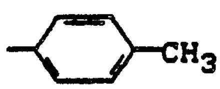 | 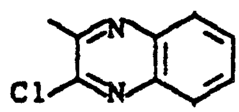 |
| $CH_3O-CH_2-$ | H | 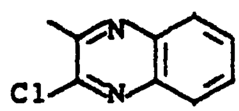 |

| $R^1$ | $R^2$ | Het |
|---|---|---|
| $CH_3O-CH_2-$ | $CH_3$ | 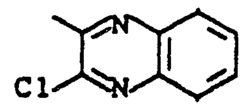 |
| $CH_3O-CH_2-$ | 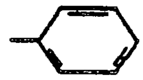 | 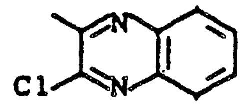 |
| $CH_3O-CH_2-$ | 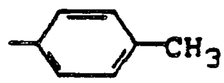 | 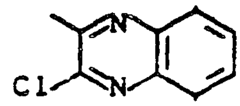 |
| 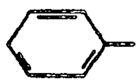 | H | 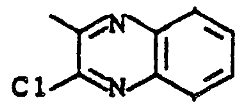 |
| 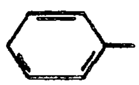 | $CH_3$ | 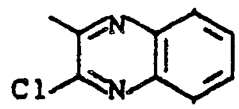 |
| 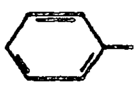 | 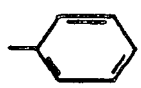 | 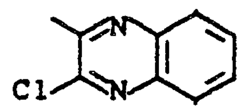 |
| 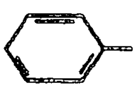 | 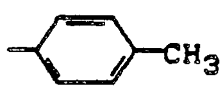 | 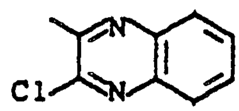 |

Verwendet man beispielsweise 4-Hydroximino-1,3-dimethyl-pyrazolin-5-on und 2,4,5,6-Tetrachlorpyrimidin als Ausgangsstoffe, so läßt sich der Reaktionsablauf des erfindungsgemäßen Verfahrens (a) durch das folgende Formelschema darstellen:

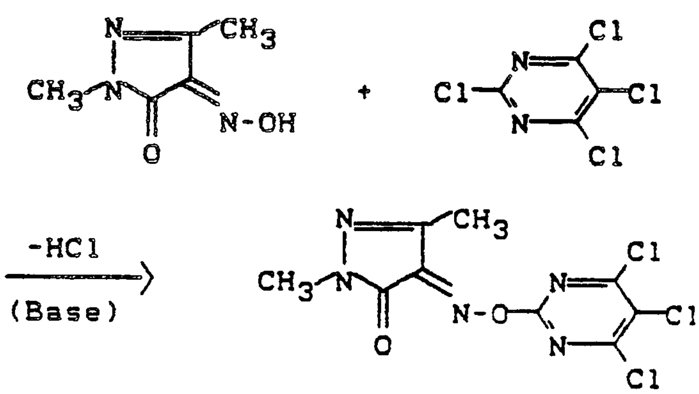


Verwendet man beispielsweise ß-Keto-α-[(3-Chlor-1,2,4-oxadiazol-5-yl)-oximino]-buttersäureethylester und Phenylhydrazin als Ausgangsstoffe, so läßt sich der Reaktionsablauf des erfindungsgemäßen Verfahrens (b) durch das folgende Formelschema darstellen:

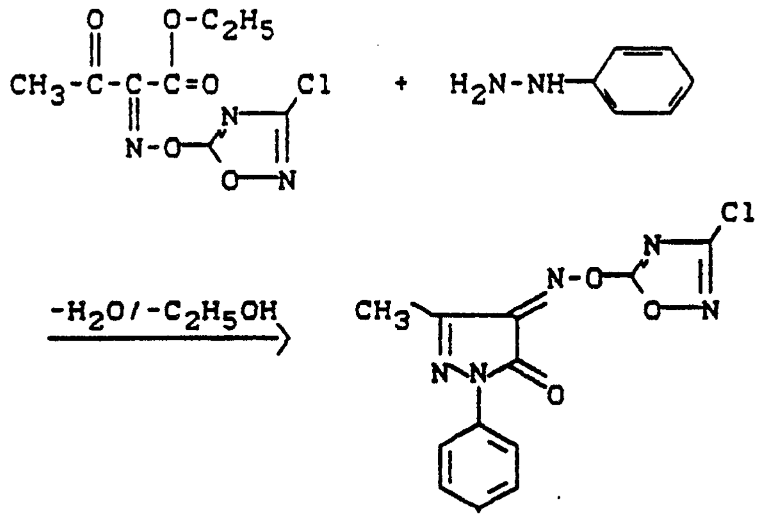


Verwendet man beispielsweise 3-Methyl-4-[(5-trifluormethylpyrimidin-2-yl)-oximino]-pyrazolin-5-on und Chloracetonitril als Ausgangsstoffe, so läßt sich der Reaktionsablauf des erfindungsgemäßen Verfahrens (c) durch das folgende Formelschema darstellen:

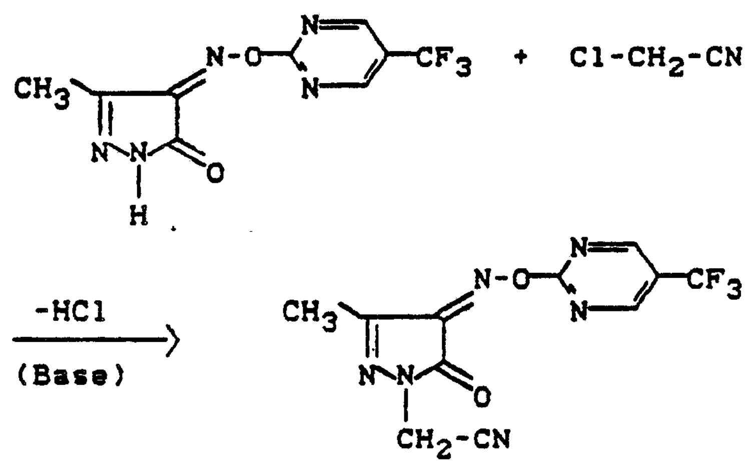


Die zur Durchführung des erfindungsgemäßen Verfahrens (a) als Ausgangsstoffe benötigten 4-Oximino-pyrazolin-5-one sind durch die Formel (II) allgemein definiert. In dieser Formel (II) stehen $R^1$ und $R^2$ vorzugsweise für diejenigen Reste, die schon bei der Beschreibung der erfindungsgemäßen Stoffe der Formel (I) als bevorzugt für diese Substituenten genannt wurden. A steht vorzugsweise für Wasserstoff oder für ein Natrium- oder Kaliumkation.

Die 4-Oximino-pyrazolin-5-one der Formel (II) sind teilweise bekannt [vgl. z.B. Ber. dtsch. chem. Ges. 29, 249 (1896); Coll. Czech. Chem. Commun. 25, 55 (1960); Arch. Pharm. 309, 900 (1976); Liebigs Ann. Chem. 1976, 1380].

Man erhält sie beispielsweise, wenn man ß-Ketoester der Formel (VII),

$$R^1-\underset{\underset{O}{\|}}{C}-CH_2-COOR^4 \qquad (VII)$$

in welcher

$R^1$ die oben angegebene Bedeutung hat und

$R^4$ für niederes Alkyl, insbesondere für Methyl oder Ethyl steht,

oder wenn man Ethoxymethylenmalonester der Formel (VIII)

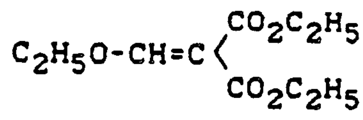

(VIII)

zunächst in einer 1. Stufe mit Hydrazinen der Formel (V),

$R^2$-NH-$NH_2$ (V)

in welcher

$R^2$ die oben abgegebene Bedeutung hat,

gegebenenfalls in Gegenwart eines Verdünnungsmittels, wie beispielsweise Ethanol, bei Temperaturen zwischen 0 °C und 100 °C cyclisiert, die aus dem Malonester der Formel (VIII) sich ergebenden 4-Ethoxycarbonylpyrazolin-5-one der Formel (IX),

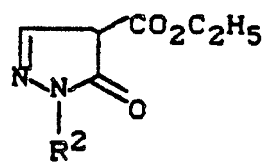

(IX)

in welcher

$R^2$ die oben angegebene Bedeutung hat,

in einem Zwischenschritt, nach üblichen Methoden, beispielsweise mit wäßriger Salzsäure bei Temperaturen zwischen 50 °C und 120 °C verseift und decarboxyliert, und die so erhältlichen Pyrazolin-5-one der Formel (X),

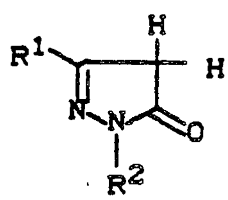

(X)

in welcher

$R^1$ und $R^2$ die oben angegebene Bedeutung haben, in einer 2. Stufe (bzw. 3. Stufe) mit einem Nitrosierungsmittel, wie beispielsweise Isopentylnitrit oder Na triumnitrit, gegebenenfalls in Gegenwart eines Verdünnungsmittels, wie beispielsweise Ethanol, Wasser oder wäßrige Salzsäure und gegebenenfalls in Gegenwart einer Base, wie beispielsweise Natriummethylat, bei Temperaturen zwischen -20 °C und +50 °C umsetzt.

Die zur Durchführung des erfindungsgemäßen Verfahrens (a) als Ausgangsstoffe benötigten Heterocyclen sind durch die Formel (III) allgemein definiert. In dieser Formel (III) steht Het vorzugsweise für diejenigen Reste, die bereits im Zusammenhang mit der Beschreibung der erfindungsgemäßen Stoffe der Formel (I) als bevorzugt für diese Substituenten genannt wurden. $E^1$ steht vorzugsweise für Halogen, insbesondere Chlor, Brom oder Iod, oder für gegebenenfalls substituiertes Alkylsulfonyloxy, Alkoxysulfonyloxy oder Arylsulfonyloxy wie beispielsweise Methansulfonyloxy, Methoxysulfonyloxy, Trifluormethansulfonyloxy oder p-Toluolsulfonyloxy.

Die Heterocylen der Formel (III) sind allgemein bekannte Verbindungen der organischen Chemie.

Die ß-Ketoester der Formel (VII) bzw. der Ethoxymethylenmalonester der Formel (VIII) sind ebenfalls allgemein bekannt.

Die zur Durchführung des erfindungsgemäßen Verfahrens (b) als Ausgangsstoffe benötigten Alkoximinocarbonsäureester sind durch die Formel (IV) allgemein definiert. In dieser Formel (IV) stehen $R^1$ und Het vorzugsweise für diejenigen Reste, die bereits im Zusammenhang mit der Beschreibung der erfindungsgemäßen Stoffe der Formel (I) als bevorzugt für diese Substituenten genannt wurden. R steht vorzugsweise für geradkettiges oder verzweigtes Alkyl mit 1 bis 4 Kohlenstoffatomen, insbesondere für Methyl oder Ethyl.

Die Alkoximinocarbonsäureester der Formel (IV) sind teilweise bekannt (vgl. z.B. EP 210 815; JP 61/143

379; JP 61/171 464; JP 61/85 392; EP 147 181; EP 76 452 oder J. chem. Soc. Perkin Trans. I, 1984, 653-656).

Man erhält sie, wenn man Hydroximinocarbonsäureester der Formel (XI),

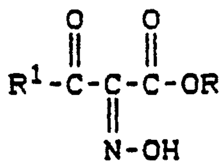

(XI)

in welcher

R und $R^1$ die oben angegebene Bedeutung haben,

mit Heterocyclen der Formel (III),

Het-$E^1$ (III)

in welcher

Het die oben angegebene Bedeutung hat und

$E^1$ für eine elektronenanziehende Abgangsgruppe, insbesondere für Chlor, Brom oder Iod, oder für gegebenenfalls substituiertes Alkylsulfonyloxy, Alkoxysulfonyloxy oder Arylsulfonyloxy wie beispielsweise Methansulfonyloxy, Methoxysulfonyloxy, Trifluormethansulfonyloxy oder p-Toluolsulfonyloxy steht,

gegebenenfalls in Gegenwart eines Verdünnungsmittels, wie beispielsweise Acetonitril sowie gegebenenfalls in Gegenwart eines Säurebindemittels, wie beispielsweise Triethylamin, bei Temperaturen zwischen +10 °C und +80 °C umsetzt.

Die Hydroximinocarbonsäureester der Formel (XI) sind allgemein bekannte Verbindungen der organischen Chemie (vgl. z.B. Helv. Chim. Acta 67, 906-915 [1984]; Franz. Patentanmeldung FR 2 434 572; Yakugaku Zasshi 87, 1209-1211 [1967] oder Chem. Ber. 100, 1245-1247 [1967]).

Die zur Durchführung des erfindungsgemäßen Verfahrens (c) als Ausgangsstoffe benötigten 4-Alkoximinopyrazolin-5-one sind durch die Formel (Ia) allgemein definiert. In dieser Formel (Ia) stehen $R^1$ und Het vorzugsweise für diejenigen Reste, die bereits im Zusammenhang mit der Beschreibung der erfindungsgemäßen Stoffe der Formel (I) für diese Substituenten genannt wurden.

Die 4-Alkoximinopyrazolin-5-one der Formel (Ia) sind erfindungsgemäße Verbindungen und erhältlich mit Hilfe der erfindungsgemäßen Verfahren (a) oder (b).

Die zur Durchführung des erfindungsgemäßen Verfahrens (c) weiterhin als Ausgangsstoffe benötigten Alkylierungsmittel sind durch die Formel (VI) allgemein definiert. In dieser Formel (VI) steht $R^2$ vorzugsweise für diejenigen Reste, die bereits im Zusammenhang mit der Beschreibung der erfindungsgemäßen Stoffe der Formel (I) für den Substituenten $R^2$ genannt wurden,mit Ausnahme des Wasserstoffrestes und der unsubstituierten oder substituierten Arylreste. $E^2$ steht vorzugsweise für diejenigen Abgangsgruppen, die bereits bei der Beschreibung der Heterocyclen der Formel (III) für den Substituenten $E^1$ genannt wurden.

Die Alkylierungsmittel der Formel (VI) sind ebenfalls allgemein bekannte Verbindungen der organischen Chemie.

Als Verdünnungsmittel zur Durchführung des erfindungsgemäßen Verfahrens (a) kommen inerte organische Lösungsmittel oder wäßrige Systeme in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder diethylether; Ketone, wie Aceton oder Butanon; Nitrile, wie Acetonitril oder Propionitril; Amide, wie Dimethylformamid, Dimethylacetamid, N-Methylformanilid, N-Methylpyrrolidon oder Hexamethylphosphorsäuretriamid; Ester, wie Essigsäureethylester; Sulfoxide, wie Dimethylsulfoxid oder auch Wasser oder wäßrig-organische Zweiphasen-Gemische, wie Dichlormethan-Wasser oder Toluol-Wasser.

Das erfindungsgemäße Verfahren (a) wird gegebenenfalls in Gegenwart eines Säurebindemittels durchgeführt. Als solche kommen alle üblichen anorganischen oder organischen Basen in Frage. Hierzu gehören beispielsweise Alkalimetallhydroxide, -amide, -alkoholate oder -hydride, wie Natriumhydroxid oder Kaliumhydroxid, Natriummethylat oder Kalium-t-butylat, Natriumhydrid oder Natriumamid; Alkalimetallcarbonate, wie Natriumcarbonat, Kaliumcarbonat oder Natriumhydrogencarbonat sowie tertiäre Amine, wie Triethylamin, N,N-Dimethylanilin, Pyridin, N,N-Dimethylaminopyridin, Diazabicyclooctan (DABCO), Diazabicyclononen (DEN) oder Diazabicycloundecen (DBU).

Die Reaktionstemperatur kann bei der Durchführung des erfindungsgemäßen Verfahrens (a) in einem größeren Bereich variiert werden. Im allgemeinen arbeitet man bei Temperaturen zwischen -20 °C und +200 °C, vorzugsweise bei Temperaturen zwischen 0 °C und +150 °C.

Bei der Durchführung des erfindungsgemäßen Verfahrens (a) setzt man pro Mol 4-Oximino-pyrazolin-5-on der Formel (II) im allgemeinen 1,0 bis 3,0 Mol, vorzugsweise 1,0 bis 1,5 Mol, an Heterocyclus der Formel (III) und gegebenenfalls 1,0 bis 3,0 Mol, vorzugsweise 1,0 bis 1,5 Mol, Säurebindemittel ein.

Führt man die Umsetzung in einem organisch-wäßrigen Zweiphasensystem durch, kann man gegebenenfalls in Gegenwart von 0,01 bis 1 Mol eines geeigneten Phasentransferkatalysators wie beispielsweise einer quartären Ammonium- oder Phosphoniumverbindung arbeiten. Beispielhaft seien Triethylbenzylammoniumchlorid und Benzyl-dodecyldimethylammoniumchlorid genannt.

Die Aufarbeitung und Isolierung der Reaktionsprodukte der Formel (I) erfolgt nach üblichen Methoden.

Als Verdünnungsmittel zur Durchführung des erfindungsgemäßen Verfahrens (b) kommmen ebenfalls inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether oder Alkohole, wie Methanol, Ethanol oder Propanol.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (b) setzt man pro Mol Alkoximinocarbonsäureester der Formel (IV) im allgemeinen 0,8 bis 2,5 Mol, vorzugsweise 1,0 bis 1,2 Mol, an Hydrazin-Derivat der Formel (V) ein. Die Reaktionsdurchführung, Aufarbeitung und Isolierung der Reaktionsprodukte der Formel (I) erfolgt nach üblichen Methoden.

Als Verdünnungsmittel zur Durchführung des erfindungsgemäßen Verfahrens (c) kommen ebenfalls inerte organische Lösungsmittel oder wäßrige Systeme in Frage.

Vorzugsweise verwendet man die bei Verfahren (a) angegebenen organischen Lösungsmittel oder wäßrigorganischen Zweiphasen-Gemische.

Das erfindungsgemäße Verfahren (c) wird gegebenenfalls in Gegenwart eines Säurebindemittels durchgeführt. Vorzugsweise verwendet man als solche die bei Verfahren (a) angegebenen anorganischen oder organischen Basen.

Die Reaktionstemperaturen können bei der Durchführung des erfindungsgemäßen Verfahrens (c) ebenfalls in einem größeren Bereich variiert werden. Im allgemeinen arbeitet man bei Temperaturen zwischen -20 °C und +200 °C, vorzugsweise bei Temperaturen zwischen 0 °C und +150 °C.

Bei der Durchführung des erfindungsgemäßen Verfahrens (c) setzt man pro Mol 4-Alkoximino-pyrazolin-5-on der Formel (Ia) im allgemeinen 1,0 bis 3,0 Mol, vorzugsweise 1,0 bis 1,5 Mol, Alkylierungsmittel der Formel (VI) und gegebenenfalls 0,5 bis 3,0 Mol, vorzugsweise 0,6 bis 1,5 Mol, Säurebindemittel ein.

Die Reaktionsdurchführung, Aufarbeitung und Isolierung der Reaktionsprodukte der Formel (I) erfolgt, wie bei Verfahren (a) beschrieben bzw. nach allgemein üblichen Verfahren.

Die erfindungsgemäßen Wirkstoffe weisen eine starke Wirkung gegen Schädlinge auf und können zur Bekämpfung von unerwünschten Schädlingen praktisch eingesetzt werden. Die Wirkstoffe sind für den Gebrauch als Pflanzenschutzmittel insbesondere als Fungizide geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deteromycetes.

Beispielhaft aber nicht begrenzend seien einige Erreger von pilzlichen Erkrankungen, die unter die oben aufgezählten Oberbegriffe fallen, genannt:

Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);

Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocerco-sporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei können die erfindungsgemäßen Wirkstoffe mit besonders gutem Erfolg zur Bekämpfung von Reiskrankheiten, wie beispielsweise gegen den Erreger der Reisfleckenkrankheit (Pyricularia oryzae), oder zur Bekämpfung von Krankheiten im Obst- und Gemüseanbau, wie beispielsweise gegen den Erreger des Apfelschorfes (Venturia inaequalis) oder gegen Cercospora-Arten eingesetzt werden. Dabei zeigen die erfindungsgemäßen Wirkstoffe neben protektiver Wirksamkeit auch systemische Eigenschaften.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt-und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenak tiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstof fen vorliegen wie Fungizide, Insektizide, Akarizide und Her bizide sowie in Mischungen mit Düngemitteln und Wachstumsregulatoren.

Die Wirkstoffe können als solche, in Form ihrer Formulierungen oder den daraus bereiteten Anwendungsformen wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g benötigt.

Bei Behandlung des Bodens sind Wirkstoffkonzentrationen von 0,00001 bis 0,1 Gew.-%, vorzugsweise von 0,0001 bis 0,02 % am Wirkungsort erforderlich.

## Herstellungsbeispiele

### Beispiel 1

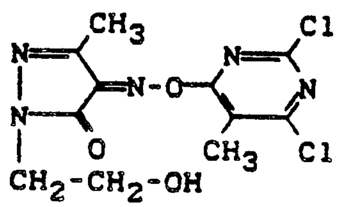

Zu 2,94 g (0,0172 Mol) 1-(2-Hydroxyethyl)-4-hydroximino-3-methylpyrazolin-5-on und 3,4 g (0,0172 Mol) 5-Methyl-2,4,6-trichlorpyrimidin (vgl. z.B. EP 126 711) in 100 ml Aceton gibt man 2,89 g (0,0344 Mol) Natriumhydrogencarbonat und 10 ml Wasser, rührt 15 Stunden bei Raumtemperatur, engt dann im Vakuum ein, nimmt den Rückstand in Dichlormethan auf, wäscht mit Wasser, trocknet über Natriumsulfat, engt wiederum im Vakuum ein und reinigt den Rückstand durch Verrühren mit Ethanol.

Man erhält 3,0 g (53 % der Theorie) an 4-[(2,6-Dichlor-5-methylpyrimidin-4-yl)-oximino]-1-(2-hydroxyethyl)-3-methylpyrazolin-5-on vom Schmelzpunkt 120 °C.

### Herstellung der Ausgangsverbindung

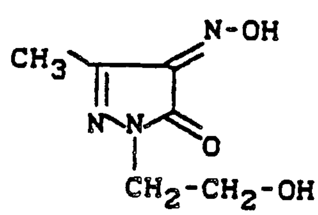

Zu 110,5 g (0,8 Mol) 1-(2-Hydroxyethyl)-3-methyl-4H-pyrazolin-5-on in 600 ml Wasser gibt man unter Eiskühlung zunächst 100 ml (1 Mol) konzentrierte Salzsäure und anschließend portionsweise 55,2 g (0,8 Mol) Natriumnitrit zu, rührt nach beendeter Zugabe 3 Stunden bei Raumtemperatur, saugt den ausgefallenen Niederschlag ab, wäscht mit Wasser nach und trocknet ihn.

Man erhält 122 g (89,2 % der Theorie) an 4-Hydroximino-1-(2-hydroxyethyl)-3-methyl-pyrazolin-5-on

vom Schmelzpunkt 166 °C.

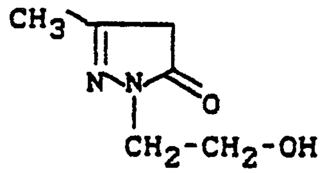

Zu 260 g (2 Mol) Acetessigsäureethylester in 250 ml Ethanol gibt man tropfenweise unter Rühren 152 g (2 Mol) 2-Hydroxyethylhydrazin, wobei die Temperatur auf 75 °C ansteigt. Nach beendeter Zugabe rührt man 15 Stunden bei Raumtemperatur, engt dann im Vakuum ein und kristallisiert das zurückbleibende Öl durch Verreiben mit Ether.

Man erhält 213 g (77 % der Theorie) an 1-(2-Hydroxyethyl)-3-methyl-4H-pyrazolin-5-on.

$^1$H-NMR ($CDCl_3$/Tetramethylsilan): $\delta$ = 2,13 ppm.

In entsprechender Weise und gemäß den allgemeinen Angaben zur Herstellung erhält man die folgenden substituierten 4-Heterocyclyloximino-pyrazolin-5-one der allgemeinen Formel (I):

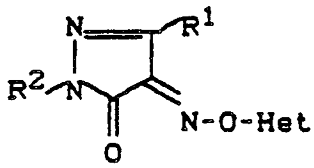

(I)

| Bsp. Nr. | $R^1$ | $R^2$ | Het | physikalische Eigenschaften |
|---|---|---|---|---|
| 2 | H | $CH_3$ | 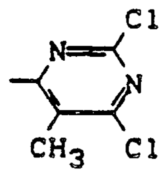 | Fp. 161 °C |
| 3 | H | H | 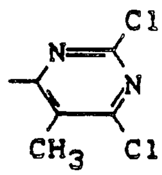 | Fp. 175-178° C (Zers.) |
| 4 | $CH_3$ | $CH_3$ | 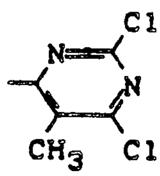 | Fp. 162-164° C |
| 5 | $CH_3$ | $CH_3$ | 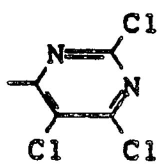 | Fp. 129-130° C |

| Bsp. Nr. | $R^1$ | $R^2$ | Het | physikalische Eigenschaften |
|---|---|---|---|---|
| 6 | $CH_3$ | $CH_3$ | 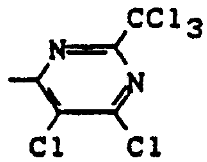 | Fp. 105-107° C |
| 7 | $CH_3$ | 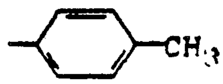 | 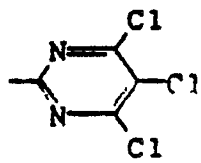 | Fp. 163-165° C |
| 8 | $CH_3$ | 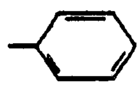 | 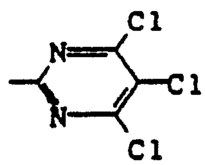 | Fp. 163-165° C |
| 9 | $CH_3$ | 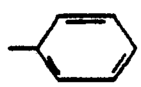 | 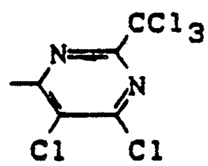 | Fp. 179-181° C |
| 10 | $CH_3$ | 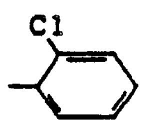 | 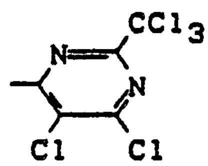 | Fp. 178-181° C |
| 11 | H | 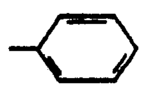 | 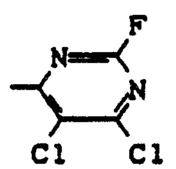 | Fp. 164-166° C |
| 12 | 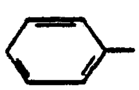 | $CH_3$ | 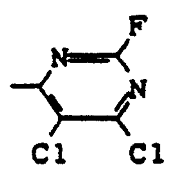 | Fp. 153-155° C |

| Bsp. Nr. | $R^1$ | $R^2$ | Het | physikalische Eigenschaften |
|---|---|---|---|---|
| 13 | 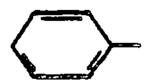 | $CH_3$ | 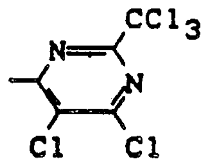 | Fp. 161-163° C |
| 14 | $C_2H_5OOC-$ | $CH_3$ | 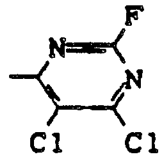 | Fp. 148-151° C |
| 15 | $C_2H_5OOC-$ | $CH_3$ | 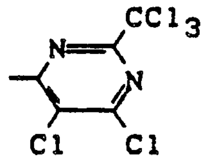 | Fp. 161-163° C |
| 16 | $CH_3O-CH_2-$ | $CH_3$ | 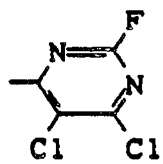 | Fp. 121-123° C |
| 17 | $CH_3$ | 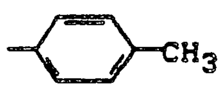 | 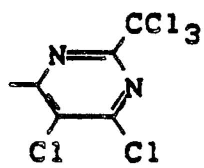 | Fp. 179-181° C |
| 18 | $CH_3$ | 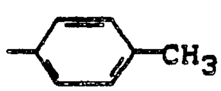 | 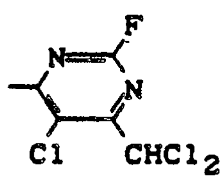 | Fp. 173-175° C |
| 19 | $CH_3$ | 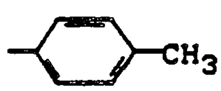 | 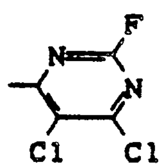 | Fp. 163-165° C |

| Bsp. Nr. | $R^1$ | $R^2$ | Het | physikalische Eigenschaften |
|---|---|---|---|---|
| 20 | $CH_3$ | 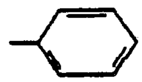 | 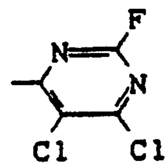 | Fp. 163-165° C |
| 21 | $CH_3$ | $CH_3$ | 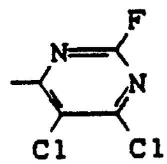 | Fp. 153-155° C |
| 22 | $CH_3$ | $CH_3$ | 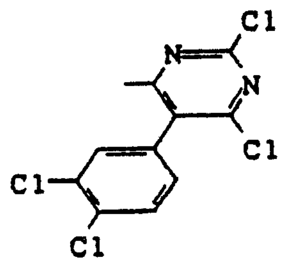 | Fp. 210-211° C |
| 23 | $CH_3$ | $CH_3$ | 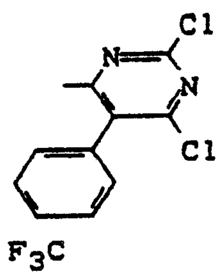 | Fp. 201-202° C |
| 24 | $CH_3$ | $CH_3$ | 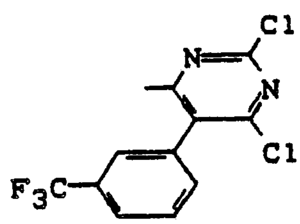 | Fp. 143-145° C |
| 25 | $CH_3$ | $CH_3$ | 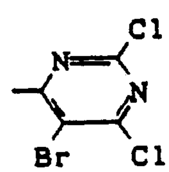 | Fp. 154-155° C |
| 26 | $CH_3$ | $CH_3$ | 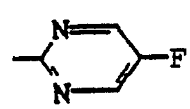 | Fp. 138-141° C |

Anwendungsbeispiele

In den folgenden Anwendungsbeispielen wurde die nachstehend aufgeführte Verbindung als Vergleichsubstanz eingesetzt:

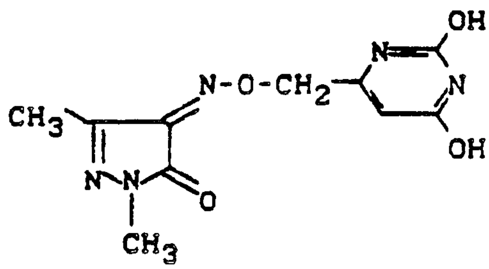

(A)

4-[(2,6-Dihydroxypyrimidin-4-yl)-methoximino]-1,3-dimethylpyrazolin-5-on

(bekannt aus EP 212 360).

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 °C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeipielen: 1, 7, 8, 10, 11, 16, 19 und 20.

Beispiel B

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25 °C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß den Herstellungsbeipielen: 7, 8, 10, 11, 16, 19, 20.

**Ansprüche**

1. Substituierte 4-Heterocyclyloximino-pyrazolin-5-one der allgemeinen Formel (I),

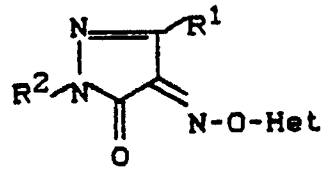

(I)

in welcher
$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils unsubstituiertes oder jeweils substituiertes Oxiranylalkyl, Aralkyl, Heterocyclyl oder Aryl stehen und
Het für einen unsubstituierten oder substituierten Heterocyclus steht,
deren geometrische Isomeren und Isomerengemische.

2. Substituierte 4-Heterocyclyloximino-pyrazolin-5-one gemäß Anspruch 1, wobei in der Formel (I),
$R^1$ und $R^2$ unabhängig voneinander jeweils für Wasserstoff, für jeweils geradkettiges oder verzweigtes Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl oder Dialkylaminocarbonylalkyl mit jeweils bis zu 8 Kohlenstoffatomen in den einzelnen Alkyl- bzw. Alkenyl- oder Alkinylteilen, für Oxiranylalkyl mit 1 bis 4 Kohlenstoffatomen im geradkettigen oder verzweigten Alkylteil, für 1,1-Dioxotetrahydrothienyl, für unsubstituiertes oder einfach bis mehrfach, gleich oder verschieden substituiertes Aralkyl mit 1 bis 4 Kohlenstoffatomen im geradkettigen oder verzweigten Alkylteil und 6 bis 10 Kohlenstoffatomen im Arylteil oder für unsubstituiertes oder einfach bis mehrfach, gleich oder verschieden substituiertes Aryl mit 6 bis 10 Kohlenstoffatomen stehen, wobei als Substituenten in den Arylteilen jeweils in Frage kommen: Halogen, Cyano, Nitro, Hydroxy, jeweils geradkettiges oder verzweigtes Alkyl, Alkoxy, Dioxyalkylen, Alkylcarbonyloxy und Alkylthio mit jeweils bis zu 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen oder Phenyl und
Het für einen unsubstituierten oder einfach bis mehrfach, gleich oder verschieden substituierten und/oder benzannellierten, gesättigten oder ungesättigten fünfgliedrigen oder sechsgliedrigen Heterocyclus mit 1 bis 3 gleichen oder verschiedenen Heteroatomen bzw. Heterogruppierungen steht, wobei als Substituenten infrage kommen: Hydroxy, Halogen, Cyano, Nitro, Amino, jeweils geradkettiges oder verzweigtes Alkyl, Alkoxy oder Alkylthio mit jeweils 1 bis 4 Kohlenstoffatomen, jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, jeweils geradkettiges oder verzweigtes Alkylamino, Dialkylamino, Alkoxycarbonyl oder Alkoximinoalkyl mit jeweils 1 bis 4 Kohlenstoffatomen in den einzelnen Alkylteilen oder unsubstituiertes oder einfach bis mehrfach, gleich oder verschieden durch Nitro, Halogen und/oder jeweils geradkettiges oder verzweigtes Alkyl oder Alkoxy mit jeweils mit 1 bis 4 Kohlenstoffatomen substituiertes Phenyl.

3. Substituierte 4-Heterocyclyloximino-pyrazolin-5-one gemäß Anspruch 1, wobei in der Formel (I)
$R^1$ und $R^2$ unabhängig voneinander jeweils für Wasserstoff, Methyl, Ethyl, n- oder i-Propyl, n-, i-, s- oder t-Butyl, Allyl, Butenyl, Propargyl, Cyanmethyl, Cyanethyl, Hydroxymethyl, Hydroxyethyl, Methoxymethyl, Methoxyethyl, Ethoxymethyl, Ethoxyethyl, Methylthiomethyl, Methoxycarbonyl, Ethoxycarbonyl, Hydroxycarbonylmethyl, Hydroxycarbonylethyl, Methoxycarbonylmethyl, Methoxycarbonylethyl, Ethoxycarbonylmethyl, Ethoxycarbonylethyl, Aminocarbonylmethyl, Methylaminocarbonylmethyl, Ethylaminocarbonylmethyl, Dimethylaminocarbonylmethyl, Diethylaminocarbonylmethyl, Aminocarbonylethyl Oxiranylmethyl, Oxiranylethyl, für 1,1-Dioxotetrahydrothien-3-yl oder für unsubstituiertes oder jeweils ein- bis dreifach, gleich oder verschieden substituiertes Phenyl oder Benzyl stehen, wobei als Phenyl-Substituenten jeweils in Frage kommen: Fluor, Chlor, Brom, Iod, Cyano, Nitro, Hydroxy, Methyl, Ethyl, n-oder i-Propyl, n-, i-, s- oder t-Butyl, Methoxy, Ethoxy, Dioxymethylen, Dioxyethylen, Methylthio, Ethylthio, Acetoxy oder Propionyloxy, Chlormethyl, Dichlormethyl, Trichlormethyl, Trifluormethyl, Dichlorfluormethyl, Chlordifluormethyl, Trifluormethoxy, Trifluormethylthio oder Phenyl und
Het für einen unsubstituierten oder ein- bis dreifach, gleich oder verschieden substituierten und/ oder benzannellierten über ein Kohlenstoffatom verknüpften Heterocyclus der Formel

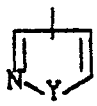 ; 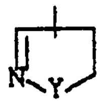 ; 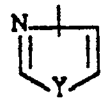 ; 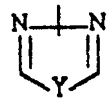 ;

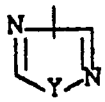 ; 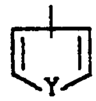 ; 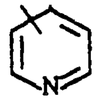 ; 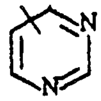 ;

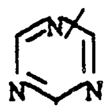 oder 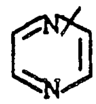 steht,

wobei als Substitutenten jeweils in Frage kommen: Hydroxy, Amino, Cyano, Nitro, Fluor, Chlor, Brom, Methyl, Ethyl, n-oder i-Propyl, n-, i-, s- oder t-Butyl, Methoxy, Ethoxy, Methylthio, Ethylthio, Trifluormethyl, Trichlormethyl, Dichlormethyl, Chlormethyl, Difluormethyl, Fluormethyl, Trifluormethoxy, Difluormethoxy, Trifluormethylthio, Methylamino, Ethylamino, Dimethylamino, Diethylamino, Methoxycarbonyl, Ethoxycarbonyl, Methoximinomethyl, Ethoximinomethyl, Methoximinoethyl, Ethoximinoethyl oder unsubstituiertes oder ein- bis dreifach, gleich oder verschieden durch Chlor, Nitro, Methyl, Ethyl und/oder Methoxy substituiertes Phenyl und wobei

Y jeweils für Sauerstoff, Schwefel, Sulfinyl oder Sulfonyl steht.

4. Substituierte 4-Heterocyclyloximino-pyrazolin-5-one gemäß Anspruch 1, wobei in der Formel (I)

$R^1$ und $R^2$ unabhängig voneinander jeweils für Wasserstoff, Methyl, Ethyl, n- oder i-Propyl, für Methoxymethyl, für Hydroxyethyl, für Methoxycarbonyl oder Ethoxycarbonyl oder für unsubstituiertes oder einfach oder zweifach, gleich oder verschieden durch Chlor, Nitro, Methyl, Ethyl und/oder Methoxy substituiertes Phenyl stehen und

Het für einen unsubstituierten oder ein- bis dreifach, gleich oder verschieden substituierten Heterocyclus der Formel

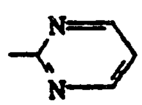 ; 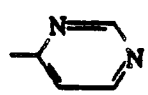 oder 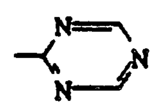 steht,

wobei als Substituenten jeweils infrage kommen: Hydroxy, Amino, Cyano, Nitro, Fluor, Chlor, Brom, Methyl, Ethyl, Methoxy, Ethoxy, Methylthio, Ethylthio, Trifluormethyl, Trichlormethyl, Dichlormethyl, Chlormethyl, Methylamino, Ethylamino, Dimethylamino, Diethylamino oder unsubstituiertes oder einfach oder zweifach, gleich oder verschieden durch Chlor, Nitro, Methyl, Ethyl und/oder Methoxy substituiertes Phenyl.

5. Verfahren zur Herstellung von substituierten 4-Heterocyclyloximino-pyrazolin-5-onen der allgemeinen Formel (I),

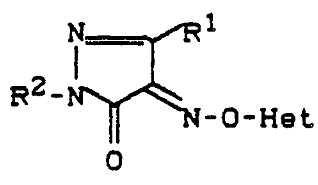 (I)

in welcher

$R^1$ und $R^2$ unabhängig voneinander jeweils für Wasserstoff, Alkyl, Alkenyl, Alkinyl, Cyanalkyl, Hydroxyalkyl, Alkoxyalkyl, Alkylthioalkyl, Alkoxycarbonyl, Hydroxycarbonylalkyl, Alkoxycarbonylalkyl, Aminocarbonylalkyl, Alkylaminocarbonylalkyl, Dialkylaminocarbonylalkyl oder für jeweils unsubstituiertes oder jeweils substituiertes Oxiranylalkyl, Aralkyl, Heterocyclyl oder Aryl stehen und

Het für einen unsubstituierten oder substituierten Heterocyclus steht,

dadurch gekennzeichnet, daß man

(a) 4-Oximino-pyrazolin-5-one der Formel (II),

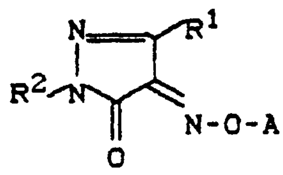

(II)

in welcher
$R^1$ und $R^2$ die oben angegebene Bedeutung haben und
A für Wasserstoff oder ein Alkalimetallkation steht,
mit Heterocyclen der Formel (III),
Het-$E^1$ (III)
in welcher
Het die oben angegebene Bedeutung hat und
$E^1$ für eine elektronenanziehende Abgangsgruppe steht,
gegebenenfalls in Gegenwart eines Verdünnungsmittels, gegebenenfalls in Gegenwart eines Reaktionshilfsmittels und gegebenenfalls in Gegenwart eines Katalysators umsetzt, oder

(b) Alkoximinocarbonsäureester der Formel (IV),

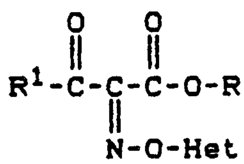

(IV)

in welcher
R für Alkyl steht und
$R^1$ und Het die oben angegebene Bedeutung haben,
mit Hydrazin-Derivaten der Formel (V),
$R^2$-NH-$NH_2$ (V)
in welcher
$R^2$ die oben angegebene Bedeutung hat,
gegebenenfalls in Gegenwart eines Verdünnungsmittels umsetzt,
oder daß man

(c) die nach Verfahren (a) oder nach Verfahren (b) erhältlichen 4-Alkoximino-pyrazolin-5-one der Formel (Ia),

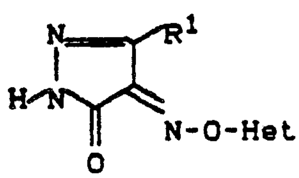

(Ia)

in welcher
$R^1$ und Het die oben angegebene Bedeutung haben,
mit Alkylierungsmitteln der Formel (VI),
$R^2$-$E^2$ (VI)
in welcher
$R^2$ für Alkyl, Alkenyl, Alkinyl, Cyanalkyl, Hydroxyalkyl, Alkoxyalkyl, Alkylthioalkyl, Hydroxycarbonylalkyl, Alkoxycarbonylalkyl oder für jeweils unsubstituiertes oder jeweils substituiertes Aralkyl oder Heterocyclyl steht und
$E^2$ für eine elektronenanziehende Abgangsgruppe steht,
gegebenenfalls in Gegenwart eines Verdünnungsmittels, gegebenenfalls in Gegenwart eines Säurebindemittels und gegebenenfalls in Gegenwart eines Katalysators umsetzt.

6. Schädlingsbekämpfungsmittel, gekennzeichnet durch einen Gehalt an mindestens einem substituierten 4-Heterocyclyloximino-pyrazolin-5-on der Formel (I) nach einem der Ansprüche 1 bis 5.

7. Verwendung von substituierten 4-Heterocyclyloximino-pyrazolin-5-onen der Formel (I) nach einem der Ansprüche 1 bis 5 zur Bekämpfung von Schädlingen.

8. Verfahren zur Bekämpfung von Schädlingen, dadurch gekennzeichnet, daß man mindestens ein substituiertes 4-Heterocyclyloximino-pyrazolin-5-on der Formel (I) nach einem der Ansprüche 1 bis 5 auf Schädlinge und/oder ihren Lebensraum einwirken läßt.

9. Verfahren zur Herstellung von Schädlingsbekämpfungsmitteln, dadurch gekennzeichnet, daß man substituierte 4-Heterocyclyloximino-pyrazolin-5-one der Formel (I) nach einem der Ansprüche 1 bis 5 mit Streckmitteln und/oder oberflächenaktiven Mitteln vermischt.

10. Verwendung von substituierten 4-Heterocyclyloximino-pyrazolin-5-onen der Formel (I) gemäß Anspruch 7 zur Bekämpfung von pflanzenpathogenen Pilzen.

Europäisches Patentamt

# EUROPÄISCHER RECHERCHENBERICHT

Nummer der Anmeldung

EP 89116259.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,A | EP - A1 - 0 212 360 (BAYER) * Ansprüche 1,3,5,6 * -- | 1-10 | C 07 D 403/12<br>A 01 N 43/48<br>A 01 N 43/56 |
| A | US - A - 4 666 933 (JELICH) * Ansprüche 1,10,11 * ---- | 1-10 | |

RECHERCHIERTE SACHGEBIETE (Int. Cl.4)

C 07 D 403/00

Der vorliegende Recherchenbericht wurde für alle Patentansprüche erstellt.

| Recherchenort | Abschlußdatum der Recherche | Prüfer |
|---|---|---|
| WIEN | 23-11-1989 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument